# EUROPÄISCHE PATENTSCHRIFT

(11) **EP 2 129 403 B1**
(45) Veröffentlichungstag und Bekanntmachung des Hinweises auf die Patenterteilung: **10.04.2013**
(21) Anmeldenummer: 08715930.7
(22) Anmeldetag: 21.02.2008
(51) Int. Cl.: A61K 47/48, A61P 9/00

(54) **Bispezifisches polypeptidisches Konstrukt mit therapeutischem und diagnostischem Potential**
Bispecific polypeptidic construct having therapeutic and diagnostic potential
Construction polypeptidique bispécifique au potentiel thérapeutique et diagnostique

(30) Priorität: 22.02.2007 DE 102007010306
(43) Veröffentlichungstag der Anmeldung: 09.12.2009
(73) Patentinhaber: Eberhard-Karls-Universität Tübingen, 72076 Tübingen (DE)
(72) Erfinder: LANGER, Harald, 72074 Tuebingen (DE); GAWAZ, Meinrad, 72076 Tuebingen (DE); BUHRING, Hans-Jorg, 72076 Tuebingen (DE); SKUTELLA, Thomas, 72070 Tuebingen (DE); JUNG, Gundram, 72108 Rottenburg-Wendelsheim (DE)
(74) Vertreter: Laufer, Gabriele
(86) Internationale Anmeldenummer: PCT/EP2008/001369
(87) Internationale Veröffentlichungsnummer: WO 2008/101700

(56) Entgegenhaltungen:
- EP-A1- 1 314 780
- WO-A1-00/06195
- LANGER H ET AL: "Adherent platelets recruit and induce differentiation of murine embryonic endothelial progenitor cells to mature endothelial cells in vitro" CIRCULATION RESEARCH, Bd. 98, Nr. 2, Februar 2006 (2006-02), Seiten e2-e10, XP002524825 US ISSN: 0009-7330
- DAUB KARIN ET AL: "Platelets induce differentiation of human CD34+ progenitor cells into foam cells and endothelial cells." THE FASEB JOURNAL : OFFICIAL PUBLICATION OF THE FEDERATION OF AMERICAN SOCIETIES FOR EXPERIMENTAL BIOLOGY, Bd. 20, Nr. 14, Dezember 2006 (2006-12), Seiten E1935-E1944, XP002524547 ISSN: 1530-6860
- LEV ELI I ET AL: "Potential role of activated platelets in homing of human endothelial progenitor cells to subendothelial matrix." THROMBOSIS AND HAEMOSTASIS, Bd. 96, Nr. 4, Oktober 2006 (2006-10), Seiten 498-504, XP008105491 ISSN: 0340-6245
- MASSBERG STEFFEN ET AL: "Platelets secrete stromal cell-derived factor 1alpha and recruit bone marrow-derived progenitor cells to arterial thrombi in vivo." THE JOURNAL OF EXPERIMENTAL MEDICINE, Bd. 203, Nr. 5, 15. Mai 2006 (2006-05-15), Seiten 1221-1233, XP002524548 ISSN: 0022-1007
- ISHIKAWA TETSUYA ET AL: "Establishment of a functionally active collagen-binding vascular endothelial growth factor fusion protein in situ." ARTERIOSCLEROSIS, THROMBOSIS AND VASCULAR BIOLOGY, Bd. 26, Nr. 9, September 2006 (2006-09), Seiten 1998-2004, XP002524549 ISSN: 1524-4636
- DUMONT B ET AL: "Chimeric Fc Receptors Identify Ligand Binding Regions in Human Glycoprotein VI" JOURNAL OF MOLECULAR BIOLOGY, Bd. 361, Nr. 5, 1. September 2006 (2006-09-01), Seiten 877-887, XP024951330 LONDON, GB ISSN: 0022-2836 [gefunden am 2006-09-01]
- MASSBERG STEFFEN ET AL: "Soluble glycoprotein VI dimer inhibits platelet adhesion and aggregation to the injured vessel wall in vivo." THE FASEB JOURNAL: OFFICIAL PUBLICATION OF THE FEDERATION OF AMERICAN SOCIETIES FOR EXPERIMENTAL BIOLOGY, Bd. 18, Nr. 2, Februar 2004 (2004-02), Seiten 397-399, XP002524551 ISSN: 1530-6860

## Beschreibung

Die Erfindung betrifft ein bispezifisches Konstrukt bzw. Fusionsprotein mit therapeutischem und diagnostischem Potenzial zur Behandlung/Diagnose von Läsionen von Gefäßen oder Geweben; ferner betrifft die Erfindung ein dieses Konstrukt bzw. Fusionsprotein codierendes Nucleinsäuremolekül, eine pharmazeutische und diagnostische Zusammensetzung, die das Konstrukt bzw. Fusionsprotein oder Nucleinsäuremolekül aufweist, sowie die Verwendung des Konstruktes bzw. Fusionsproteins oder Nucleinsäuremoleküls zur Herstellung einer pharmazeutischen Zusammensetzung zur Behandlung von Läsionen von Gefäßen/Geweben sowie zur Therapie von akuten oder chronischen Gefäßerkrankungen.

Verletzungen der Gefäße des Herz-Kreislaufsystems treten insbesondere als Folge von Stent- oder Stentgraftimplantationen in die Gefäße auf, welche wiederum aufgrund anderer Erkrankungen oder Vorfälle in die betroffenen Gefäße eingeführt werden müssen, um die Versorgung des umliegenden Gewebes oder von Organen zu gewährleisten.

Im physiologischen Zustand zirkuliert das Blut in einem geschlossenen Gefäßsystem, ohne dass es zum Anhalten des Blutflusses oder zum Austritt von Blut in umliegendes Gewebe kommt. Allerdings führen Schäden in der Gefäßwand zur Aufhebung der Integrität der Gefäßwand und zur anschließenden Blutung in umliegendes Gewebe. Um dies zu verhindern, bilden Thrombozyten in Verbindung mit löslichen Plasmakomponenten einen hämostatischen Thrombus, der den Schaden abdichtet und die Blutstillung bewirkt. Sobald eine Läsion an einem Gefäß auftritt, werden sofort die verschiedenen zellulären und biochemischen Mechanismen in Gang gesetzt, die für die Hämostase notwendig sind. Auch das Endothel spielt durch die Regulation der Permeabilität für Plasmalipoproteine, Leukocytenadhäsion und die Sezernierung von pro- und antithrombotischen Faktoren sowie vasoaktiven Substanzen eine zentrale Rolle bei der arteriellen Hämostase.

Das Endothel bildet die einschichtige Gefäßwandauskleidung, die den Blutstrom von den thrombogenen Strukturen des Subendothels trennt. Bei einem Endothelschaden der Gefäßwand und der nun offenliegenden thrombogenen subendothelialen Matrix kommt es im Rahmen der Hämostase zur Adhäsion ruhender, im Blut zirkulierender Thrombozyten an nun freiliegendes Kollagen. Dieser initiale Adhäsionsvorgang wird durch thrombozytäre Membranglycoprotein-Rezeptoren, die Integrine, gesteuert und resultiert in eine Formveränderung, Thrombozytenaktivierung und Freisetzung der Inhaltstoffe aus dem Speichergranula. Dabei interagiert das thrombozytäre Glycoprotein VI direkt mit dem freiliegenden Kollagen und stabilisiert die Bindung. GPVI als wichtigster Kollagenrezeptor vermittelt nicht nur eine festere Bindung direkt an Kollagen, sondern vermittelt auch die Aktivierung anderer, zur Adhäsion notwendiger Rezeptoren. Nach der Adhäsion folgt als nächster Schritt in der Hämostase die Aggregation, die zu einer Anhäufung von Thrombozyten im Thrombus führt.

Daher spielt bei der Aktivierung der Blutplättchen das Glycoprotein VI (GPVI) als Kollagenrezeptor auf der Thrombozytenoberfläche eine entscheidende Rolle und gilt auch als Risikofaktor für Myokardinfarkte. Thrombozyten ohne GPVI zeigen keine Adhäsion an Kollagen und die Aktivierbarkeit und Aggregation ist deutlich herabgesetzt.

In diesem Zusammenhang offenbaren Langer et al. ("Adherent Platelets Recruit and Induce Differentiation of Murine Embryonic Endothelial Progenitor Cells to Mature Endothelial Cells in vitro"; Circulation Research (2006) 98(2):e2-e10) und Daub et al., ("Platelets induce differentiation of human CD34+ progenitor cells into foam cells and endothelial cells"; The FASEB Journal (2006) 20(4):2006-2012), dass adhärierende Plättchen murine embryonale endotheliale Vorläuferzellen rekrutieren und deren Reifung bzw. Differenzierung in Endothelzellen induzieren können.

Auch offenbaren Lev et al. ("Potential role of activated platelets in homing of human endothelial progenitor cells to subendothelial matrix", Thrombosis and Haemostasis (2006) 96(4):498-504) Versuche, anhand derer aufgezeigt wird, dass Plättchen und endotheliale Vorläuferzellen über p-Selectin und den p-Selectin-Rezeptor interagieren.

Die EP 1 314 780 A1 offenbart Hybridproteine, die einerseits an Kollagen binden, und die andererseits ein funktionelles Polypeptid tragen. Das an Kollagen bindende Polypeptid ist dabei eine vom humanen Fibronectin abgeleitete Aminosäuresequenz und das funktionelle Polypeptid kann bspw. VEGF sein.

Schließlich offenbaren Dumont et al. ("Chimeric Fc Receptors Identify Ligand Binding Regions in Human Glycoprotein VI", J.Mol.Biol. (2006) 361:877-887) chimäre Fc Rezeptoren, über welche Liganden-bindende Regionen in menschlichem Glycoprotein VI (GPVI) identifiziert werden konnten. Die Autoren dieser Veröffentlichung haben dabei in einem löslichem GPVI-Fusionsprotein (GPVI-Fc) zwei Domänen substituiert, und festgestellt, dass die Domäne 2 eine spezifische Rolle bei der Bindung von GPVI an Kollagen spielt.

Durch das Auftreten solcher Thromben ist die Versorgung des Gewebes mit Blut nicht mehr gewährleistet, so dass ischämische Zustände des distal des Thrombus gelegenen Gewebes eintreten können.

So machen Herz-Kreislauf-Erkrankungen, wie bspw. Angina oder Myokardinfarkt, gegenwärtig immer noch ca. ein Drittel aller weltweiten Todesfälle aus. Bei diesen Erkrankungen ist eine schnelle Reperfusion der von einer Ischämie betroffenen Koronararterien von äußerster Wichtigkeit, um eine Verletzung des Myokards zu verhindern.

Sowie der Blutfluss in einem Koronargefäß vermindert wird, treten irreversible Schäden an den Myozyten auf, die den Funktionsstoffwechsel im Myokard zum Stillstand bringen, wodurch es letztendlich zum Zelluntergang durch Nekrose und Apoptose kommt.

Wie weiter oben erwähnt, wird zur Wiederherstellung bzw. Therapie des normalen Herz-Kreislaufs gegenwärtig die transluminale perkutane Angioplastie in Verbindung mit einer Stent-Implantation eingesetzt. Nach Implantation des Stents ist zwar der freie Fluss durch das Gefäß wieder gewährleistet, jedoch ist das Gefäßendothel, das zwischen den zirkulierenden Blutzellen und der subendothelialen Matrix unter physiologischen Bedingungen eine Barriere darstellt, immer noch beschädigt. Daher stellt die Anhaftung von Blutplättchen und die darauf folgende Thrombenbildung und der daraus resultierende akute Myokardinfarkt eine Hauptkomplikation nach der Stent-Implantation dar.

Bei Reperfusion des ehemals durch einen Gefäßverschluss ischämischen Bereiches wird dieser wieder mit oxygeniertem Blut versorgt, wodurch der Zellschaden zum einen begrenzt wird, wobei jedoch dieser Prozess mit einer weiterführenden Myokardschädigung verbunden ist. Zur Akuttherapie des Myokardinfarkts werden gegenwärtig interventionelle Therapieverfahren, wie perkutane transluminale koronare Angioplastie, koronare Stent-Implantation, Laser-Ablationsangioplastie, etc. oder eine medikamentöse antithrombotische Therapie, wie Thrombolyse und Fibrinolyse, eingesetzt. Beide Therapieverfahren arbeiten auf das gleiche Ziel hin, nämlich auf die möglichst schnelle Wiedereröffnung des okkludierten Gefäßes und damit auf die obligatorische Reperfusion des ischämischen Gewebes.

Da gegenwärtig auch Stents eingesetzt werden, die mit Arzneimitteln beschichtet sind, welche nach der Implantation nach und nach freigesetzt werden, wird auch durch die freigesetzten Arzneimittel die Reendothelialisierung des behandelten Gefäßes herausgezögert, so dass die Stent-Thrombose eine äußerst kritische Komplikation dieses Verfahrens darstellt.

Aufgabe der vorliegenden Erfindung ist es daher, ein neues Mittel für die Aufrechterhaltung der endothelialen Integrität zur Verhinderung einer arteriosklerotischen Plaqueerosion bereitzustellen, mit welchem die Nachteile des Standes der Technik überwunden werden können.

Gemäß der vorliegenden Erfindung wird diese Aufgabe durch ein bispezifisches Konstrukt bzw. Fusionsprotein gelöst, das (a) ein erstes Polypeptid aufweist, das an Kollagen bindet, und (b) ein zweites Polypeptid, das an endotheliale Vorläuferzellen bindet, wobei das erste Polypeptid der Kollagenrezeptor thrombozytäres Glycoprotein (GPVI) ist, oder funktionelle Fragmente davon, und wobei das zweite Polypeptid ein Antikörper ist, der gegen CD133 gerichtet ist, oder funktionelle Fragmente davon.

Die der Erfindung zugrunde liegende Aufgabe wird auf diese Weise vollständig gelöst.

Erfindungsgemäß wird unter einem "Fusionsprotein" ein Hybridprotein bzw. ein artifizielles Protein verstanden, welches *in vitro* aber auch in *vivo* durch im Stand der Technik bekannte molekularbiologische oder chemische Verfahren herstellbar sind.

Vorläufer-(Progenitor-)zellen sind allgemein Abkömmlinge einer adulten Stammzelle, und weisen einerseits hinsichtlich ihrer Regenerationsfähigkeit Stammzelleigenschaften auf, und sind aber andererseits auf ihren künftigen Funktionsbereich festgelegt, wobei diese "Festlegung" noch umkehrbar ist. Als "endotheliale Vorläuferzellen" werden demnach im Blut zirkulierende Zellen bezeichnet, die die Fähigkeit haben, zu Endothelzellen zu differenzieren. Diese endothelialen Vorläuferzellen tragen spezifischen Zelloberflächenproteine und können daher wiederum über an diese Zelloberflächenproteine bindende Polypeptide eingefangen werden.

Unter "Polypeptid" wird vorliegend jede Kette von mindestens zwei miteinander verbundenen Aminosäuren verstanden; der Begriff "Polypeptid" umfasst daher auch Proteine, die ebenso wie Polypeptide aus mehreren miteinander verbundenen Aminosäuren bestehen. Als "Protein" werden manchmal auch nur vollständige Moleküle in stabiler Form bezeichnet, wohingegen "Polypeptide" oder "Peptide" kürzere Aminosäurenketten ohne eine stabile 3-dimensionale Struktur verstanden werden. Da jedoch keine eindeutige Grenze zwischen diesen Begriffen zu ziehen ist, sind vorliegend definitionsgemäß mit dem Begriff "Polypeptide" explizit auch Proteine umfasst.

So kann das Konstrukt bzw. Fusionsprotein bspw. durch Konjugation zweier (oder mehrerer) Polypeptide mittels eines oder mehrer chemischer Reagenzien oder durch rekombinante DNA-Technologien hergestellt werden. Andererseits besteht die Möglichkeit, das Fusionsprotein durch Verwendung üblicher Expressionsvektoren zu generieren, die für das erfindungsgemäße Fusionsprotein codieren. Diese Expressionsvektoren werden in eine geeignete Zelle eingebracht, die dann das Fusionsprotein produziert.

Die Erfinder der vorliegenden Anmeldung konnten in eigenen Versuchen zeigen, dass mit den erfindungsgemäßen Fusionsproteinen CD34⁺-Stammzellen an freigelegte Kollagenoberflächen rekrutiert werden können. Ferner konnten die Erfinder der vorliegenden Anmeldung zeigen, dass durch die Rekrutierung der Stammzellen an das exponierte Kollagen nach einer bestimmten Zeit die Stammzellen in reife Endothelzellen gereift werden konnten, was zu einer Reendothelialisierung des verletzten Gewebes führte.

Mit den erfindungsgemäßen Konstrukten bzw. Fusionsproteinen ist es danach möglich, auf einfache und gezielte Weise die Reendothelialisierung und die Wiederherstellung von verletzten Gefäßen bzw. von jeglichem Gewebe, das durch eine Verletzung oder sonstige Einflüsse auf seiner Oberfläche Kollagen freigibt bzw. exponiert, durch die Besiedelung mit Stammzellen und deren Reifung in Endothelzellen therapiert werden kann. Dadurch ist es möglich, die durch eine Stent-Implantation oder durch chemische Agenzien hervorgerufenen gefäßschädigenden Reaktionen zu verhindern, bzw. nach deren Auftreten erfolgreich zu behandeln.

Zur Zeit sind etwas 27 verschiedene Kollagene bekannt. Sie machen mit bis zu einem Viertel des Gesamtproteingewichts den größten Anteil an Proteinen im Körper aus. Kollagene setzen sich aus jeweils drei gleichen oder unterschiedlichen alpha-Ketten zusammen, die fest umeinander gewunden sind.

Endothel-Vorläuferzellen stellen eine zirkulierende, Knochenmarks-abgeleitete Zellpopulation großer nicht-leukozytärer Zellen dar, die offenbar bei der Gefäßreparatur und bei der Hämostase beteiligt sind. Mit dem erfindungsgemäßen bispezifischen Konstrukt konnten unter fließenden Bedingungen Stammzellen an verletzte menschliche Gewebe rekrutiert werden.

Ein Vorteil des Konstruktes bzw. Fusionsproteins ist ferner, dass die Substanz bspw. direkt über einen Ballonkatheter verabreicht werden kann, oder mit einer Stammzellpopulation koinkubiert werden kann, bevor diese Zellen verabreicht werden, ohne dass eine schwierige und teure Beschichtung der Koronarstents vonnöten wäre. Das vorliegende Fusionsprotein stellt daher ein äußerst wirksames Werkzeug dar, mit welchem Stammzellen an verletzte Gefäßläsionen rekrutiert werden können, und stellt daher ein wirksames therapeutisches Konzept für die Behandlung von arterosklerotischen Krankheiten dar.

Wie bereits eingangs erwähnt, stellt der Rezeptor GPVI den wichtigsten Rezeptor der Thrombozyten für Kollagen dar. GPVI ermöglicht die Aggregation, Sekretion, Formveränderung und Aktivierung der Blutplättchen. Humanes GPVI enthält eine Signalsequenz mit 20 Aminosäuren, eine extrazelluläre Domäne von 247 Aminosäuren, sowie eine 21 Aminosäuren lange Transmembran-Domäne und einen 51 Aminosäure langen cytoplasmatischen Schwanz.

Das erfindungsgemäße Konstrukt bwz. Fusionsprotein bindet mit dem genannten Rezeptor bzw. Rezeptor-Fragmenten als erstes Polypeptid an exponiertes Kollagen. Über das zweite Polypeptid, welches in dem Konstrukt bzw. Fusionsprotein enthalten ist, werden endotheliale Vorläuferzellen, also bestimmte Stammzellen, an das exponierte Kollagen rekrutiert, und zwar dadurch, dass die endothelialen Vorläuferzellen durch das Oberflächenantigen CD133 an das das Antigen erkennende Polypeptid des Fusionsproteins bzw. Konstrukts binden. Die derart rekrutierten Stammzellen reifen nach einer bestimmten Inkubationszeit zu Endothelzellen und können dadurch das verletzte Gewebe regenerieren, wodurch Kollagen nicht mehr freigelegt und daher nicht mehr thrombozytär ist.

In einer Ausführungsform ist bevorzugt, wenn das erste Polypeptid einen extrazellulären Anteil von GPVI, oder funktionelle Fragmente davon, verbunden mit einer Immunglobulin-Fc-Domäne aufweist.

Hierbei ist von Vorteil, dass bspw. auf bereits lösliches GPVI zurückgegriffen werden kann, das zuvor im Stand der Technik beschrieben wurde, siehe Massberg et al., "Soluble glycoprotein VI dimer inhibits platelet adhesion and aggregation to the injured vessel wall in vivo", FASEB J. 2004; 18: 397-399, wobei bezüglich der Herstellung von löslichem humanem GPVI auf diese Veröffentlichung explizit Bezug genommen wird. Lösliches GPVI zeigt nur als dimere Form in Verbund mit der Immunglobulin-Fc-Domäne Affinität zu Kollagen. Zur Generierung dieses löslichen GPVI wurde der extrazelluläre Anteil des humanen GPVI kloniert und mit der humanen Immunglobulin-Fc-Domäne verbunden. Dieses GPVI-Fc-Protein (im Folgenden lösliches GPVI-FC genannt) kann bspw. mit Hilfe von Adenoviren über eine humane HeLa-Zelllinie exprimiert werden. Mit diesem löslichen GPVI-Fc konnte sowohl in *vitro* als auch *in vivo* die Adhäsion an Kollagen nachgewiesen werden.

Es versteht sich für einen Fachmann, dass zur Erfüllung der erfindungsgemäßen Funktion das Fusionsprotein nicht zwingend die vollständige bzw. identische Aminosäuresequenz des löslichen GPVI eingesetzt werden muss. Vielmehr wird die erfindungsgemäße Funktion des Fusionsproteins auch dann erfüllt, wenn das erste Polypeptid einen Abschnitt oder eine Sequenzvariante des löslichen GPVI aufweist, der bzw. die jedoch noch die Bindefunktion von GPVI in ggf. abgeschwächter Form ausübt. Bekanntermaßen werden die proteinogenen Aminosäuren in vier Gruppen unterteilt, nämlich in polare, nicht-polare, saure und basische Aminosäuren. Der Austausch einer polaren Aminosäure gegen eine andere polare Aminosäure, bspw. Glycin gegen Serin, führt in der Regel zu keiner oder nur einer geringfügigen Änderung der biologischen Aktivität des entsprechenden Proteins, so dass ein solcher Aminosäureaustausch das erfindungsgemäße Fusionsprotein in seiner Funktion weitgehend unberührt lässt. Vor diesem Hintergrund erfasst die vorliegende Erfindung auch ein solches Fusionsprotein, das als erstes Polypeptid eine Variante von löslichem GPVI, bei der eine oder mehrere Aminosäuren einer der genannten Aminosäure-Klassen gegen eine andere Aminosäure der selben Klasse ausgetauscht ist. Eine solche Sequenzvariante ist dabei vorzugsweise zu ca. 70 %, weiter vorzugsweise zu ca. 80 % und höchst vorzugsweise zu ca. 90 bis 95 % homolog zu der Aminosäuresequenz von löslichem GPVI.

"Fc" steht für "fragment crystallizable". Dieses Fragment entsteht durch Papain-Spaltung des IgG-Moleküls neben den beiden Fab-Fragmenten. Die Fc-Domäne besteht aus den gepaarten C_{H}2- und C_{H}3-Domänen einschließlich der Gelenkregion (engl. "hinge region") und enthält den Teil des Immunglobulins, der für die Dimerisierungsfunktion verantwortlich ist. Vorteilhafterweise kann hierbei auf käuflich erhältliche humane - oder Maus - Fc-DNA zurückgegriffen werden, die entweder aus käuflich erhältlichen cDNA-Bibliotheken durch PCR isoliert werden kann, oder die bereits in Plasmide kloniert vorliegt, welche wiederum käuflich erworben werden können (bspw. erhältlich von der Firma Invitrogen, San Diego, USA).

Es versteht sich, dass auch ein Fragment oder eine Variante der Fc-Domäne verwendet werden kann, ohne dass die erfindungsgemäße Funktion des ersten Polypeptids beeinträchtigt wird, solange das Fragment bzw. die Variante noch die ggf. abgeschwächte Dimerisierungsfunktion eines Antikörpers aufweist; vgl. vorstehende Ausführungen zum Fragment oder der Variante von GPVI, die für das Fragment oder die Variante von Fc gleichermaßen gelten.

Dabei wird vorzugsweise eine Variante der Fc-Domäne oder ein synthetisches Fc-Fragment eingesetzt, welches im Komplement- und Fc-Rezeptorbindungsbereich derart mutiert ist, dass eine Aktivierung des Immunsystems weitgehend reduziert wird und ggf. sogar ausbleibt. So kann bspw. ein Fc-Fragment eingesetzt werden, bei dem durch gezielte Mutagenese an der Position 331 ein Prolin gegen ein Serin und an den Aminosäurepositionen 234 bis 237 das Tetrapeptid Leu-Leu-Gly-Gly gegen Ala-Ala-Ala-Ala ausgetauscht wird.

In einer weiteren Ausführungsform ist bevorzugt, wenn das erste Polypeptid eine Aminosäuresequenz mit der SEQ ID Nr. 3 aus dem beigefügten Sequenzprotokoll aufweist.

Die Aminosäuresequenz SEQ ID-Nr. 3 stellt die extrazelluläre Domäne des humanen GPVI dar, dessen Gesamtsequenz in SEQ ID-Nr. 1 wiedergegeben ist.

Die Aminosäuresequenz SEQ ID-Nr. 5 stellt die extrazelluläre Domäne des humanen DDR-1 dar, dessen Gesamtsequenz in SEQ ID-Nr. 4 wiedergegeben ist, die Aminosäuresequenz SEQ ID Nr. 6 stellt die Sequenz von DDR-2 dar, dessen extrazelluläre Domäne in SEQ ID Nr. 7 dargestellt ist.

Das Fusionsprotein kann dabei ein erstes Polypeptid aufweisen, das von einem Abschnitt des Nucleinsäuremoleküls codiert wird, das die Nucleotidsequenz SEQ ID-Nr. 2 aus dem beiliegenden Protokoll aufweist.

Die Nucleotidsequenz SEQ ID-Nr. 2 stellt die für das humane GPVI codierende Nucleotidsequenz dar.

Es versteht sich, dass nicht nur die Nucleotidsequenz SEQ ID-Nr. 2 zur Herstellung der extrazellulären Domänen der Kollagen-Rezeptoren geeignet ist, sondern auch Varianten hiervon, die auf Grund der Degeneration des genetischen Codes für dasselbe Polypeptid codieren. So ist es bekannt, dass der genetische Code degeneriert ist, da die Anzahl der möglichen Codons größer ist als die Anzahl der Aminosäuren. Für die meisten Aminosäuren gibt es mehr als ein Codon, so dass bspw. Arginin, Leucin und Serin von bis zu sechs Codons codiert wird. In der Regel ist die dritte Codonposition begrenzt oder völlig austauschbar. Vor diesem Hintergrund wird auch ein solches Fusionsprotein bereitgestellt, bei dem das erste Polypeptid von einem Nucleinsäuremolekül codiert wird, das auf Grund der Degeneration des genetischen Codes gegenüber der Nucleotidsequenz SEQ ID-Nr. 2 an einzelnen Nucleotidpositionen abweicht, jedoch gleichermaßen für die extrazelluläre Domänen von GPVI codiert. Vorzugsweise weist eine solche Variante zu der Nucleotidsequenz SEQ ID-Nr. 2 ca. 70 % Homologie, weiter vorzugsweise ca. 80 % Homologie und höchst vorzugsweise ca. 90 bis 95 % Homologie auf.

Gemäß der vorliegenden Erfindung ist das zweite Polypeptid ein Antikörper, der gegen CD133 gerichtet ist, oder funktionelle Fragmente davon.

Das Antigen CD133 wird auf hämatopoetischen und endothelialen Vorläuferstammzellen sowie auf einigen epithelialen Zellen exprimiert. Das Antigen stellt demnach einen Marker für diese Stammzellen dar, und ist im Stand der Technik hinreichend beschrieben worden (siehe bspw. Yin et al., "CD133: A novel marker for human hematopoetic stem and progenitor cells", Blood (1997) 90:5002-5012). Über einen dieses Antigen erkennenden Antikörper können demnach die Stammzellen, die wiederum dieses Antigen aufweisen, durch Bindung an das zweite Polypeptid des Fusionsproteins an das Kollagen rekrutiert werden.

So kann bspw. ein anti-CD133-Antikörper oder funktionelle Fragmente davon eingesetzt werden, der gegenwärtig kommerziell erhältlich ist, wie bspw. der CD133 Antikörper von Miltenyi Biotech (Klon W6B3C1), Bergisch Gladbach, Deutschland, oder der CD133 Antikörper der Firma abcam Inc. (32AT1672), Cambridge, Großbritannien.

Der Antikörper W6B3C1 wurde durch Immunisierung von Mäusen mit der Retinoblastomzelllinie WERI-RB-1 gewonnen.

Es versteht sich, dass jeder gegen CD133 gerichtete Antikörper oder funktionelle Fragmente davon zum Zwecke der vorliegenden Erfindung eingesetzt werden kann. Unter Einsatz des entsprechenden Antigens ist es unter Anwendung der im Stand der Technik üblichen Techniken (bspw. der Hybridomtechnik, siehe Köhler und Milstein, "Continuous cultures of fused cells secreting antibody of predefined specificity", Nature (1975) 256:495-7) möglich, weitere, neue Anti-CD133 Antikörper zu generieren.

Vorliegend bedeuten "funktionelle Fragmente" eines Antikörpers jegliche Antikörperabschnitte oder -teile, die die gleiche Funktion bzw. Bindespezifität wie der gesamte Antikörper, von dem sie abgeleitet sind, aufweisen.

Es versteht sich, dass ausgehend von im Stand der Technik bekannten Maus-anti CD133 -Antikörpern auch zunächst humanisierte Antikörper gewonnen werden können, die dann im Fusionskonstrukt eingesetzt werden. Bei humanisierten Antikörpern handelt es sich um rekombinante Antikörper, bei denen die Sequenzen für die hypervariablen Regionen (CDR) in menschlichen Immunglobulin-Genen gegen die CDR von Immunglobulin-Genen der Maus ausgetauscht sind. Durch diese Humanisierung wird die Antigenspezifität eines monoklonalen Antikörpers der Maus auf einen humanen Antikörper übertragen. Dadurch kann im Empfängerorganismus eine vollständige Toleranz gegenüber diesen Molekülen erzeugt werden, wodurch eine humane-Anti-Maus-Antikörper-Antwort und vermieden wird. Solche Antikörper werden auch als chimäre Antikörper bezeichnet.

In einer anderen Ausführungsform kann bei dem Konstrukt bzw. Fusionsprotein ein weiteres Peptidelement vorgesehen sein, das das erste Polypeptid mit dem zweiten Polypeptid verbindet. Auch hierdurch können die Polypeptide über eine Brücke, bzw. einem Linker verbunden werden, wobei gleichzeitig die Funktionalität der beiden Polypeptide, das heißt, also das spezifische Erkennen der jeweiligen Bindungsstellen, erhalten bleibt.

Die Erfindung betrifft ferner eine pharmazeutische und/oder diagnostische Zusammensetzung, die ein einerfindungsgemäßes Konstrukt bzw. Fusionsprotein aufweist, sowie ggf. zumindest einen pharmazeutisch akzeptablen Träger sowie ggf. weitere pharmazeutisch und/oder diagnostisch wirksame Stoffe.

Diagnostisch und pharmazeutisch akzeptable Träger mit ggf. weiteren Zusätzen sind im Stand der Technik allgemein bekannt und werden bspw. in der Abhandlung von Kibbe A., Handbook of Pharmaceutical Excipients, Third Edition, American Pharmaceutical Association and Pharmaceutical Press 2000, beschrieben. Zusätze umfassen erfindungsgemäß jedwede Verbindung oder Zusammensetzung, die für eine diagnostische oder therapeutische Verwendung der Zusammensetzung vorteilhaft sind, worunter Salze, Bindemittel und weitere im Zusammenhang mit der Formulierung von Arzneimitteln üblicherweise verwendete Stoffe fallen.

Ferner betrifft die Erfindung die Verwendung des Konstrukts bzw. Fusionsproteins in der Behandlung von Läsionen von Gefäßen, wobei die Gefäße und/oder Gewebe ausgewählt sind aus der Gruppe umfassend Herzkranzgefäße, himversorgende Gefäße, extremitätenversorgende Gefäße, Bindegewebe, Knochen, sowie jedes Gefäß oder Gewebe, das Kollagen aufweist.

Mit einer erfindungsgemäß hergestellten Zusammensetzung, die das erfindungsgemäße Konstrukt bzw. Fusionsprotein enthält, wird ein äußerst wirksames Werkzeug zur Behandlung von Krankheiten bereitgestellt, deren Ursache die Läsion von Gefäßen bzw. Geweben ist, bei welchen als Folge thrombogenes Subendothel freiliegt, was zur Bildung von Thromben führen kann.

Dabei ist in einer Ausführungsform bevorzugt, wenn die Zusammensetzung zur Verabreichung über einen Ballon-Katheter hergestellt wird.

Alternativ ist bevorzugt, wenn die Zusammensetzung oder das Konstrukt bzw. Fusionsprotein mit einer Stammzelllösung vor der Zellverabreichung coinkubiert wird.

Dies hat den Vorteil, dass schwierige und teure Beschichtungsverfahren von Koronarstents vermieden werden.

Die Erfindung betrifft ferner ein Verfahren zur Herstellung eines Konstrukts bzw. Fusionsproteins, mit den folgenden Schritten: (a) Bereitstellen einer löslichen Form des Glycoproteins VI (GPVI) und eines gegen CD133 gerichteten Antikörpers; (b) Modifizieren der Aminogruppen von GPVI und des Antikörpers mit einem vernetzenden Agens; (c) Reduzieren von GPVI; und (d) Konjugieren des reduzierten GPVI mit dem in Schritt (b) modifizierten Antikörper.

Insbesondere ist bevorzugt, wenn bei dem erfindungsgemäßen Verfahren das vernetzende Agens SPDP N-Succinimidyl-3-(2-pyridyldithio)-propionat) ist.

Alternativ kann auch jedes andere im Stand der Technik bekannte vernetzende Agens oder Kopplungsverfahren eingesetzt werden, wie bspw. die Verbindung über Thioether-Crosslinker, oder aber über die rekombinante DNA-Technologie.

Mit dem erfindungsgemäßen Verfahren kann ein Fusionsprotein bereitgestellt werden, das für einen diagnostischen/therapeutischen Zweck oder Einsatz geeignet ist.

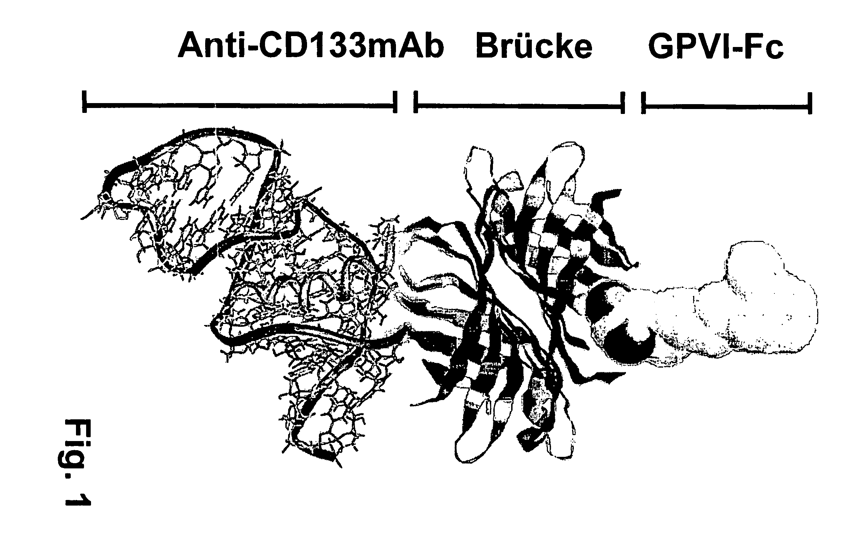

Die Erfindung wird in dem nachstehenden Beispiel und in den Figuren näher erläutert. Es zeigen
- **Fig. 1**: eine schematische Darstellung einer Ausführungsform des erfindungsgemäßen bispezifischen Konstrukts, das gegen Kollagen und das Stammzellantigen CD133 gerichtet ist.

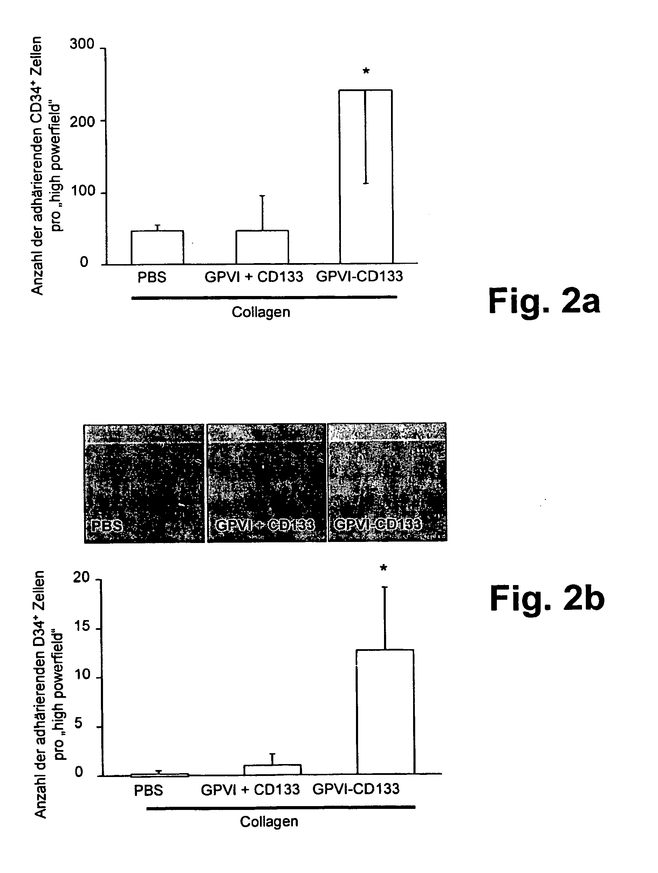

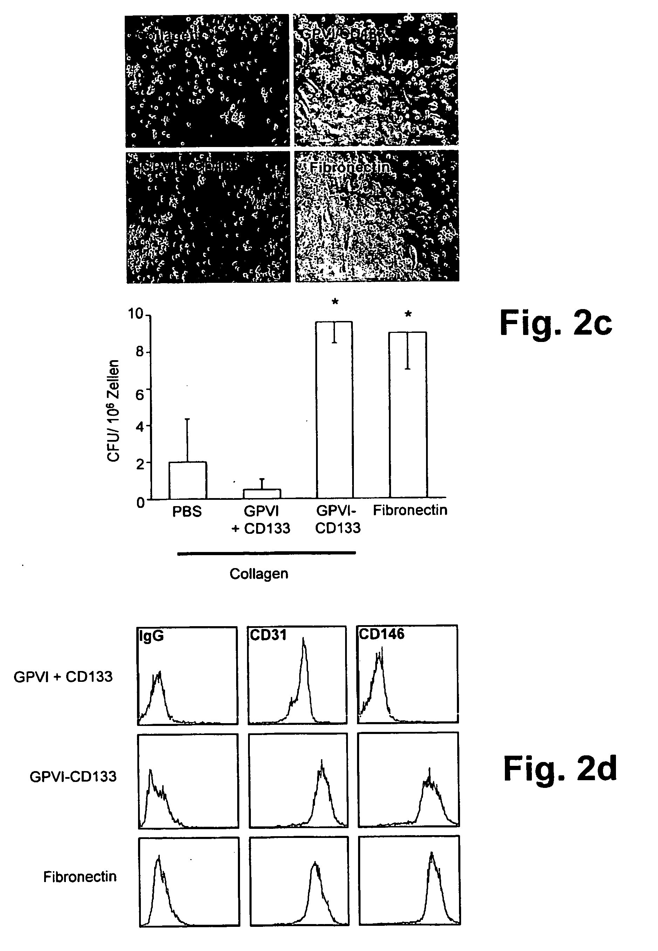

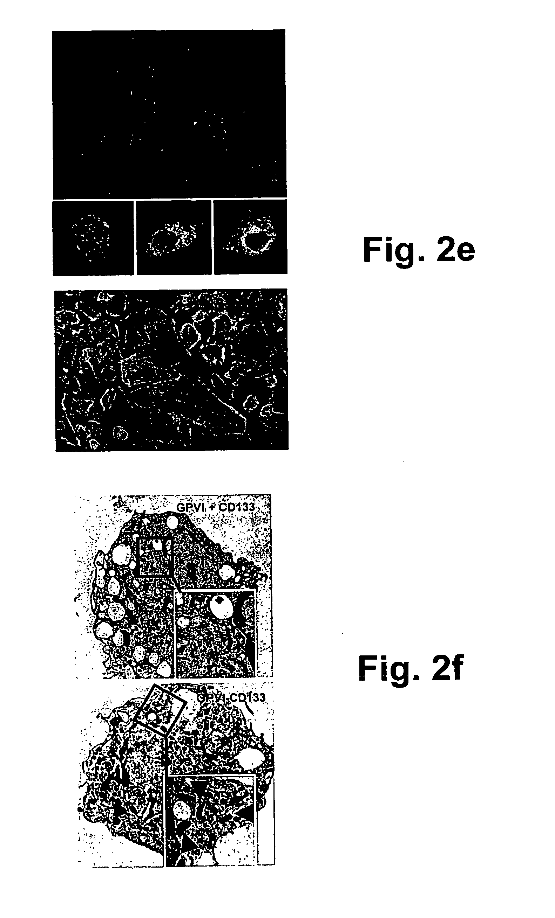

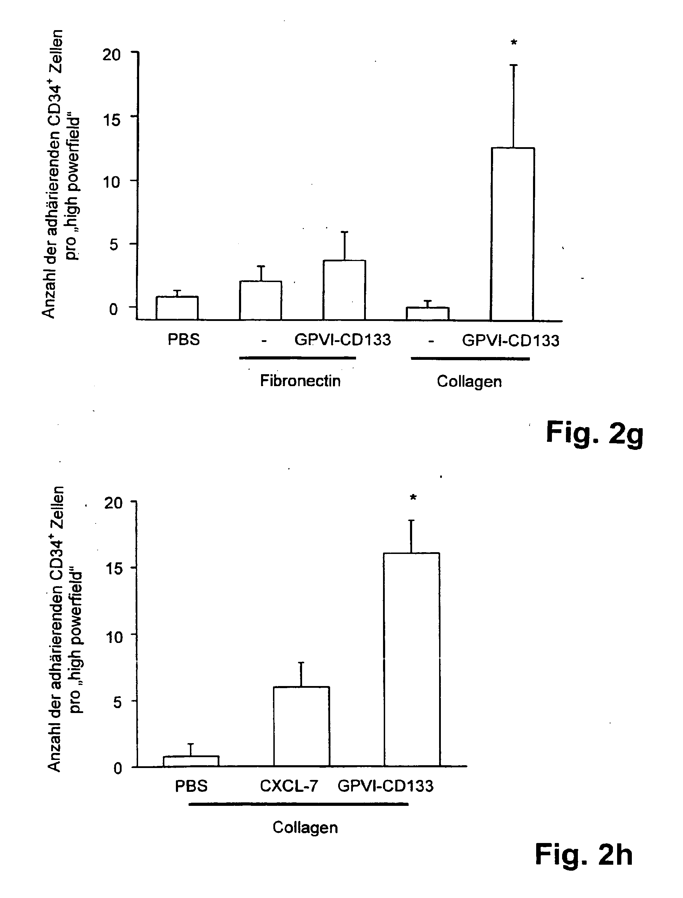

- **Fig. 2**: Die Rekrutierung von EPCs an exponiertes Kollagen durch ein spezifisches GPVI/CD133-Konstrukt, bestehend aus dem löslichen KollagenRezeptor GPVI und einem gegen CD133 gerichteten Antikörper führt zur Entwicklung von Endothelzellen *in vitro*: a) statisches Adhäsionsassay; b) dynamisches Assay; c) Bildung von endothelialen Kolonien; d) Marker-Expression von CD31 und CD146; e) Expression von vWF/Endoglin; f) Nachweis von Weibel-Palade-Körperchen im Elektronenmikroskop; g) spezifische verstärkte Adhäsion der EPCs, vermittelt durch GPVI-CD133, an immobilisiertes Kollagen im Vergleich zu Fibronectin; h) wirksamere Rekrutierung von EPCs an Kollagen durch GPVI-CD133 als CXCL7; und

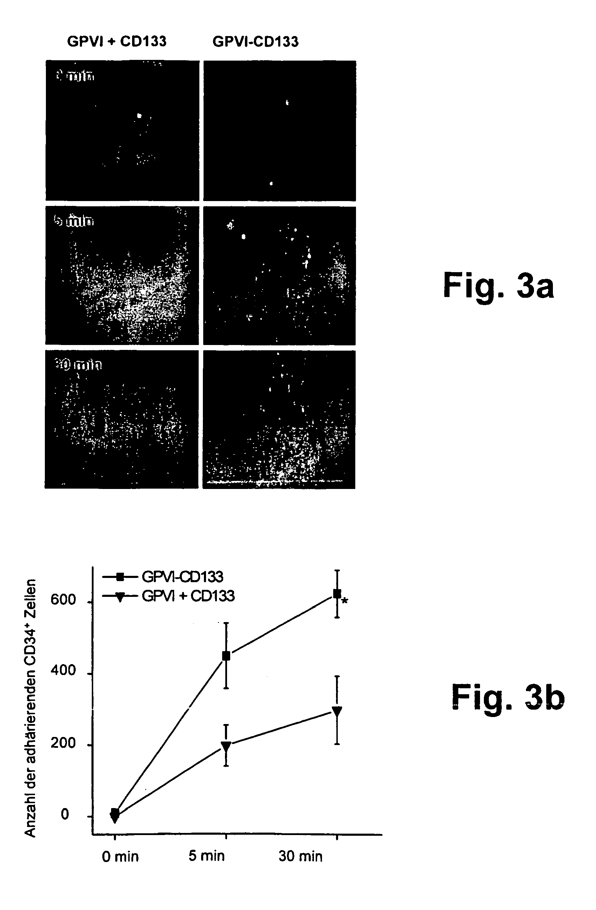

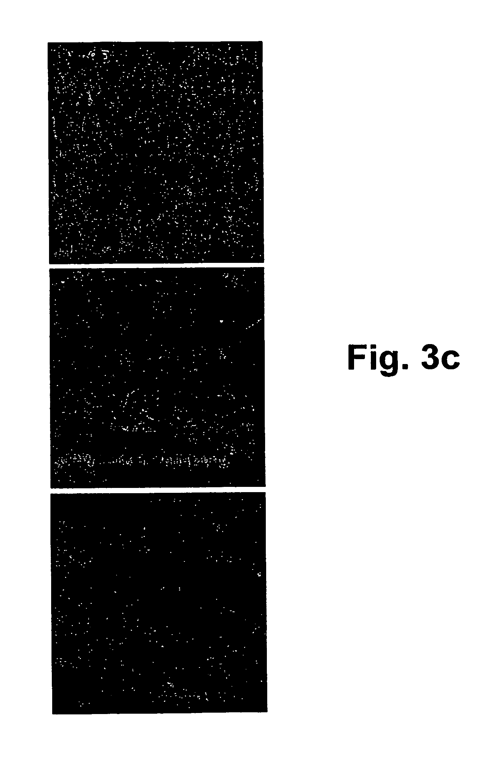

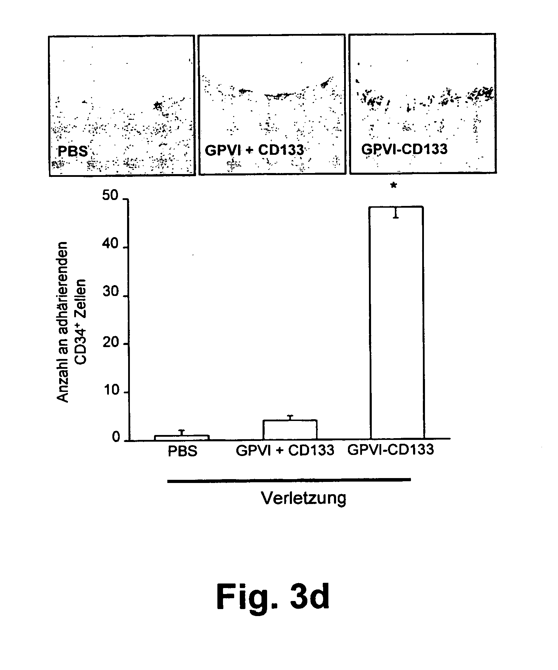

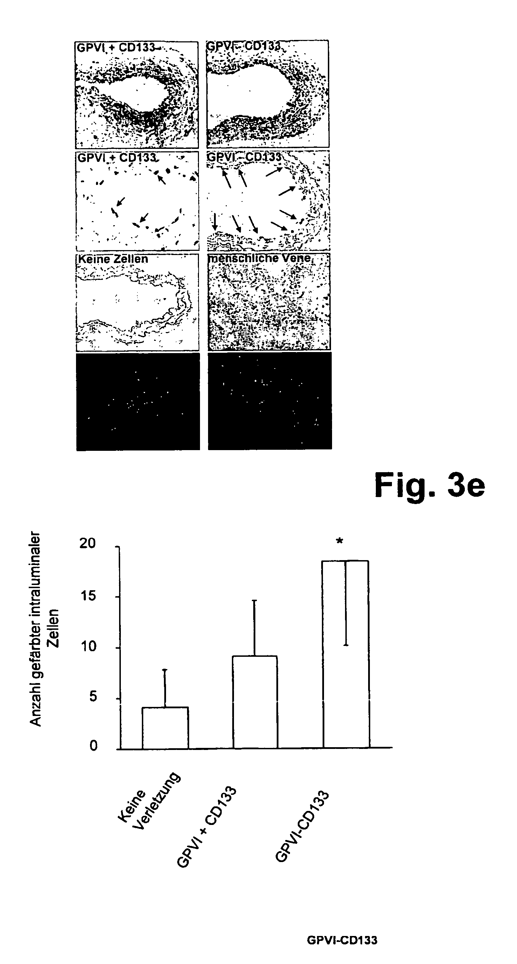

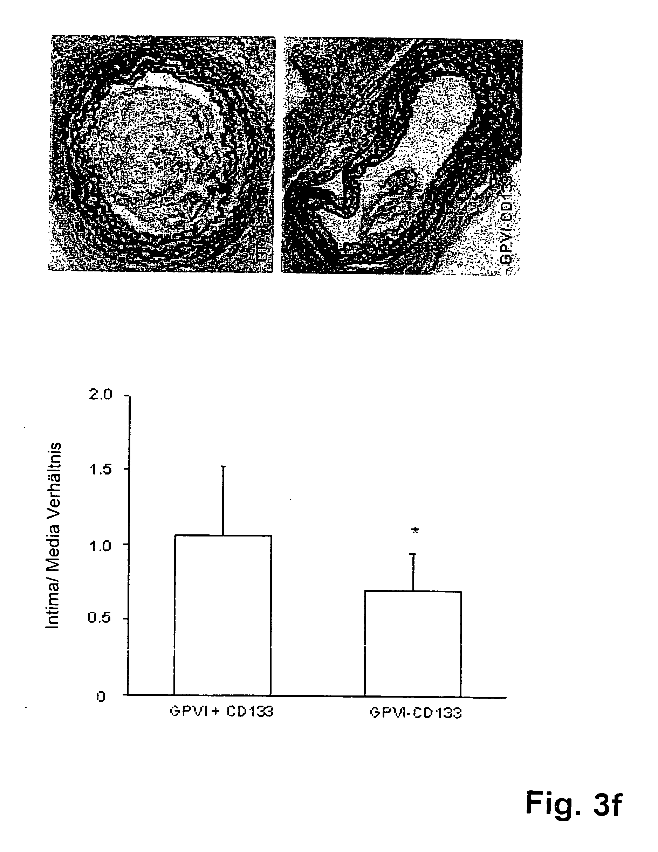

- **Fig. 3**: Das GPVI-CD133-Konstrukt rekrutiert EPCs an Gefäßläsionen *in vivo* und verstärkt die Wiederherstellung der Gefäßintegrität: **a), b)** Verletzung der Halsschlagader von Versuchstieren und Injizierung von EPCs mit DCF-Färbung; c) Histologische Schnitte, analysiert durch Zwei-Photonen-Mikroskopie und mit DCF gefärbt; **d)** *in situ* Hybridisierung histologischer Schnitte mit einer humanen alu-Sequenz; **e)** HE-Färbung von Endothelzellen; *in* situ Hybridisierung mit einer alu-Sequenz; **f)** GPVI-CD133 bewirkt ein signifikant verkleinertes Intima/Media-Verhältnis.

### Beispiel:

### Herstellung eines Bispezifischen Proteins/monoklonalen Antikörper-Konstruktes zur Rekrutierung von Knochenmarkstammzellen an Gefäßläsionen

### Material und Methoden

### Reagenzien

Biocoll-Trennungslösung wurde bei Biochrom AG (Berlin, Deutschland), EBM als "BulletKit" (EGM) von Cambrex Bio Science (East Rutherford, New Jersey) käuflich erworben. Kollagen I, Kollagen III, Laminin, Vitronectin, Fibrinogen und Fibronectin wurden von BD Sciences (Heidelberg, Deutschland), menschliches VEGF von Pepro-Tech Inc. (Rocky Hill, N.J.), der primäre Maus-Antikörper anti-vWF und das Phalloidin-AlexaFluor 488 von Chemicon (Temecula, CA) käuflich erworben. DAPI, Cy3-markierte sekundäre Antikörper (Ziegen-Anti-Maus) und das "Celltracker Vybrand DiD" wurde bei Molecular Probes/Invitrogen GmbH (Karlsruhe, Deutschland) käuflich erworben.

### Isolierung und Kultivierung von CD34⁺-Zellen und CD133⁺-Zellen

Menschliche CD34⁺-Zellen und CD133⁺-Zellen wurden aus menschlichem Nabelschnurblut isoliert und wie von Lang et al. ("Transplantation of a combination of CD133+ and CD34+ selected progenitor cells from alternative donors", British Journal of Haematology 2004; 124: 72-79) beschrieben, kultiviert. Die Spenderzellen wurden durch die Verabreichung von 1 x 10 µg/kg Granulocyten-stimulierender Faktor (G-CSF) für 5 Tage mobilisiert und durch 1 bis 3 Leukaphereseverfahren geerntet. Die Auswahl der Vorläuferzellen mit anti-CD34⁺- oder anti-CD133⁺-beschichteten Mikrokügelchen wurde mit der automatisierten CLINIMACS-Vorrichtung (Miltenyi Biotec, Bergisch Gladbach, Deutschland) durchgeführt. Vor und nach der Zellauftrennung wurden die Zellpopulationen mit Anti-CD34⁺- Anti-CD133⁺-, Anti-CD3⁺-, AntiDC19⁺- und Anti-CD45⁺-Antikörpern gefärbt und durch Fluoreszenz-aktivierte Zellsortierungsgeräte (FACS) mit FACSCalibur-Instrumenten analysiert (Becton-Dickinson, Heidelberg, Deutschland).

### Herstellung eines GPVI-CD133⁺mAB-Konstruktes

Um die Anhaftung von Stammzellen an exponiertes Kollagen zu bewirken, wurde ein bispezifisches Konstrukt (Fusionsprotein) hergestellt. Hierzu wurde lösliches GPVI-Fc und ein monoklonaler Antikörper gegen CD133 verwendet. Lösliches GPVI wurde, wie zuvor beschrieben, hergestellt, siehe hierzu Massberg *et al.,* siehe oben, wobei zur Herstellung des löslichen GPVI-Konstruktes explizit auf diese Veröffentlichung Bezug genommen wird. Kurz gesagt, wurde die extrazelluläre Domäne von GPVI an die humane Fc-Domäne fusioniert. Hierzu wurde Fc aus einer humanen Herz cDNA Bibliothek (Clontech, Palo Alto, CA, USA) amplifiziert. Die Primer Paare und die Bedingungen für die Polymerase-Ketten-Reaktion finden sich in der zitierten Veröffentlichung von Massberg et al. Das PCR-Fragment wurde über NotI/HindIII in das Plasmid pADTrack CMV kloniert. Für die Klonierung der extrazellulären Domäne von humanem GPVI wurde Gesamt-RNA aus kultivierten Megakaryozyten isoliert (RNeasy Mini Kit, Qiagen, Hilden, Deutschland). Nach einer reversen Transkription wurden 100 ng der generierten cDNA als Template für die PCR-Amplifizierung des humanen GPVI eingesetzt (Primer und PCR-Bedingungen siehe zitierte Veröffentlichung). Das PCR-Fragment wurde über BglII/NotI in das Plasmid pADTrack CMV Fc kloniert, wodurch ein Plasmid gewonnen wurde, das die humane extrazelluläre Domäne von GPVI, fusioniert an die humane Fc Domäne, einschließlich einer spezifischen Hinge-Region, enthielt.

Der CD133-reaktive monoklonale Antikörper (mAb) W6B3C1 wurde durch Immunisierung von 6 Wochen alten weiblichen Balb/c-Mäusen (Charles River WIGA, Sulzfeld, Deutschland) mit der Retinoblastomzelllinie WERI-RB-1 generiert. Die Spezifität des monoklonalen Antikörpers für CD133 wurde bei der 7. Internationalen Leukozytenkonferenz in England bestätigt (siehe Bühring et al., "CD133 Cluster Report. In: Leucocyte Typing VII. White Cell Differentiation Antigens." Mason D et al., (Eds.), Oxford University Press, Oxford, 20002, Seiten 622-623).

Zur Konjugation der beiden Proteine wurde das heterobifunktionale Reagens SPDP (N-Succinimidyl-3-(2-pyridyldithio)-propionat) gemäß dem Verfahren nach Carlsson et al., Biochem. J. 173:723 (1978), eingesetzt. Hierzu wurden die Aminogruppen der beiden Proteine mittels SPDP modifiziert. Das modifizierte GPVI-Protein wurde mit DTT (Dithiothreitol) reduziert und mit dem nicht-reduzierten, SPDP-modifiziertem CD133-Antikörper konjugiert. Die Konjugationsmischung wurde durch Gelfiltration über eine Superdex S200 Säule gereinigt.

Eine schematische Darstellung des so gewonnenen bispezifischen Konstrukts ist in Fig. 1 gezeigt.

### Statische und dynamische Adhäsionsassays

*Statische Adhäsion.* Um die Adhäsion der Vorläuferzellen an unterschiedliche extrazelluläre Matrixproteine mit oder ohne das Fusionsprodukt unter statischen Bedingungen zu bestimmen, wurden 96-well-Platten mit Kollagen I, Fibrinogen, Fibronectin oder Vitronectin (jeweils 10 µg/ml) übernacht beschichtet. In weiteren Versuchen wurden die mit Kollagen I beschichteten 96-well-Platten eine Stunde lang mit dem Fusionsprotein (10µg/ml) vorinkubiert. Als Negativkontrolle dienten die einzelnen Komponenten des Konstrukts zusammen oder die einzelnen Komponenten alleine. Anschließend wurden die Vorläuferzellen hinzugefügt und eine Stunde inkubiert. Nach drei vorsichtigen Waschschritten mit Tyrodes-Puffer wurden die verbleibenden adhärierenden Vorläuferzellen mittels Phasenkontrastmikroskopie ausgezählt.

*Dynamische Adhäsion.* Hierzu wurden Glas-Objektträger mit Kollagen I (10 µg/ml) beschichtet (siehe Langer et al., Thromb. Haemost. 2005; 94:555-561) und in einer Fließkammer eingesetzt (Oligene, Berlin, Deutschland). Anschließend wurde das Fusionsprotein (10 µg/ml) zu der Kollagenoberfläche über 30 min hinzugefügt. Als Kontrolle dienten wiederum Versuche mit den einzelnen Komponenten zusammen oder mit den einzelnen Komponenten alleine. Die Perfusion wurde mit Stammzellen durchgeführt, die in Tyrodes-HEPES Puffer (HEPES 2,5 mmol/l; NaCl 150 mmol/l; KCl 1 mmol/l; NaHCO₃ 2,5 mmol/l, NaH₂PO₄ 0,36 mmol/l; Glucose 5,5 mmol/l; BSA 1 mg/ml, pH 7,4, ergänzt mit CaCl₂ 1 mmol/l; MgCl₂ 1mmol/l; jeweils von Sigma, Taufkirchen, Deutschland) mit einer Scherrate von 2000 s-1. Sämtliche Versuche wurden in Echtzeit auf Video aufgezeichnet und off-line ausgewertet.

### Kolonie-Bildungs-Assay und Flusszytometrie

CD34⁺-Vorläuferzellen wurden auf humanem Kollagen I ausgesät unter den folgenden unterschiedliche Bedingungen: mit oder ohne Zusatz des GPVI-CD133-Konstruktes (10 µg/ml), beide einzelnen Komponenten des Konstruktes (negativ Kontrolle), Fibronectin (Becton Dickinson, Heidelberg, Deutschland) als positiv Kontrolle. Die Zellen wurden jeweils mehrere Tage in Wachstumsmedium for endotheliale Zellen MV2 mit 5 % Hitze-inaktiviertem fötalem Kälberserum, 5,0 ng/ml epidermaler Wachstumsfaktor, 0,2 µg/ml Hydrocortison, 0,5 µg/ml vaskulärer endothelialer Wachstumsfaktor , 10 ng/ml basischer Fibroblastenfaktor, 20 ng/ml R3 Insulin-ähnlicher Wachstumsfaktor-1, und 1 µg/ml Ascorbinsäure (PromoCell, Heidelberg, Deutschland) kultiviert. Nach 48 Stunden wurden die nichtadhärierenden Zellen entfernt. Endotheliale Kolonie-bildende Einheiten wurden an Tag 4 ausgezählt (Anzahl der Kolonien/10⁶ Zellen). Die Zellen wurden gewaschen und in PBS resuspendiert, für 15 min mit Polyglobin (Bayer Vital; Leverkusen, Deutschland) inkubiert, gewaschen und anschließend mit FITC-markierten Antikörpern gegen CD31 (Klon 5.6; Beckman Coulter, Krefeld Deutschland) and CD164 (Klon 128018; R&D Systems Wiesbaden, Deutschland) 30 min lang bei Raumtemperatur inkubiert. Nach einem weiteren Waschschritt wurden die Zellen mit einem FACSCanto Flusszytometer (Becton Dickinson, Heidelberg, Deutschland) analysiert.

### Transmissions-Elektronen-Mikroskopie und Immunfluoreszenz-Mikroskopie

Endotheliale Vorläuferzellen (EPC) (2 x 10⁸/ml) wurden über acht Tage in Kulturmedium MV 2 (PromoCell) in Wells inkubiert, die mit dem GPVI-CD133⁺ mAb beschichtet waren. Darüber hinaus wurden täglich Phasenkontrast-Kontrollen durchgeführt. Anschließend wurden die Zellen in Kamovsky's Lösung fixiert, in Osmiumtetroxid nachfixiert und in Glycidether eingebettet, bevor die Mikroskopie durchgeführt wurde.

Für die Immunfluoreszenz-Mikroskopie wurden die Zellen zusätzlich mit Fluoreszenzmarkierten Antikörpern inkubiert. Zwischen jedem Inkubationsschritt wurden die Zellen vorsichtig mit PBS gewaschen. Die Stammzellen wurden in 2% Formaldehydlösung 20 Minuten lang fixiert. Anschließend wurden die Zellen mit 3 % Glycin gewaschen und 30 Minuten mit PBS inkubiert, das einen primären anti-vWF Antikörper (human; 5 µg/ml) enthielt. Die unspezifische Bindung wurde mit bovinem Serum-Albumin (3%, eine Stunde) verhindert. Im Anschluss daran wurde ein sekundärer Antikörper (Ziege-Anti-Maus; 5 µg/ml) für weitere 30 Minuten hinzugefügt. Ferner wurden Rhodaminphalloidin (5 µg/ml; Detektion des Zytoskeletts) und DAPI (5 µg/ml; Detektion des Zellnukleus) 30 Minuten lang beigefügt. Die Proben wurden mittels einer Standard-Immunfluoreszenz-Mikroskopie analysiert.

### Ligatur der Halsschlagader und Untersuchung der EPC-Adhäsion durch intravitale Mikroskopie

Um den Effekt des GPVI-CD133-Konstruktes auf die Rekrutierung von Progenitorzellen *in vivo* zu untersuchen, wurde eine intravitale Mikroskopie wie bereits anderswo beschrieben (siehe Massberg et al., "A critical role of platelet adhesion in the initiation of atherosclerotic lesion formation", J. Exp. Med. 196: 887-896 (2002)) vorgenommen. Vor den Experimenten wurden die EPCs mit 5-Carboxyfluorescein-Diacetat-Succinimidylester (DCF) gefärbt und mit dem GPVI-CD133-Konstrukt (10 µg/ml) oder beiden einzelnen Komponenten des Konstrukts (jeweils 10 µg/ml) 30 min inkubiert. Wild-Typ C57BL6/J-Mäuse (Charles River Laboratories) wurden durch intraperitoneale Injektion mit Midazolam (5mg/kg Körpergewicht); Ratiopharm), Medetomidin (0,5 mg/kg Körpergewicht; Pfizer) und Fentayl (0,05 mg/kg Körpergewicht; CuraMed/Pharam GmbH) betäubt. Polyehtylen-Katheter (Portex) wurden in die rechte Jugularvene implantiert und fluoreszierende EPCs (5x10⁵/ml) intravenös injiziert. Die rechte Halsschlagader wurden freigelegt und fünf Minuten lang nahe der Karotisgabel energisch ligiert, um eine Gefäßschädigung zu induzieren. Vor und nach der Gefäßschädigung wurde die Wechselwirkung der fluoreszierenden EPCs mit der verletzten Gefäßwand durch in situ *in vivo* Video-Mikroskopie der rechten Halsschlagader sichtbar gemacht, und zwar unter Verwendung eines Zeiss Axiotech Mikroskop (20 x Wasser-Immersions-Objektiv, W 20x/0,5; Carl Zeiss MicroImaging, Inc.) mit einer 100-W HBO Quecksilberlampe für die Epi-Illumination. Gebundene EPCs wurden als Zellen definiert, die mit der Gefäßwand einen initialen Kontakt aufbauten, gefolgt von einer langsamen Oberflächen-Translokation mit einer Geschwindigkeit, die signifikant langsamer ist als die mittlere Geschwindigkeit, oder durch eine feste Adhäsion. Die Anzahl der adhärierenden EPCs wurden durch Auszählung der Zellen, die sich nicht bewegten oder sich nicht innerhalb von 10 s von der Endothel-Oberfläche ablösten, bestimmt. Ihre Anzahl wird als Zellen/mm² Endothel-Oberfläche angegeben.

### Zwei-Photonen-Mikroskopie

Die Zwei-Photonen-Mikroskopie wurde weitgehend wie bereits von van Zan et al., "Two-photon microscopy for imaging of teh artherosclerotic vascular wall: a proof of concept study", J. Vasc. Res. 41: 54-63 (2004) beschrieben, durchgeführt. Kurz gesagt wurde die Mäuse nach der intravitalen Mikroskopie getötet, die Halsschlagadern vorsichtig entfernt und mit PBS gewaschen, in Paraffin eingebettet und 4 µm-Schnitte angefertigt. Anschließend wurden die Schnitte gefärbt und mit einem BioRad 2100MP nach dem Zwei-Photonen-Laserscan-Mikroskopie-(Two-Photon laser scanning microscopy, TPLSM)-Verfahren analysiert.

### Ex vivo Untersuchung der EPC-Adhäsion an verletzte Gefäße von Schweinen

Nach der Isolierung wurden die Stammzellen mit Vybrant DiD 20 Minuten lang markiert und in EBM Medium resuspendiert. Menschliche Venen wurden in einen ex-vivo Fluss gegeben, in welchem das Gefäß aus Nährstoffgründen von Medium umgeben war. Die Gefäße wurden mittels eines Ballon-Katheters verletzt und anschließend mit dem GPVI-CD133 mAb -Konstrukt 30 Minuten lang beschichtet. Anschließend wurden EPCs zwei Stunden lang durch die Venen geführt, um den Zellen eine Adhäsion an die verletzte Gefäßregion zu ermöglichen. Um die Stabilität der Adhäsion unter natürlichem physiologischen Scherstress zu testen, wurden die Venen danach gründlich mit EBM mit einer hohen Scherrate 24 Stunden lang bei 37°C gewaschen. Im Anschluss daran wurden die Gefäße aus dem Bioreaktor entfernt, 24 Stunden lang in 4 % PFA fixiert, und die Zell-Rekrutierung durch in situ Hybridisierung analysiert.

### In vivo Untersuchung der Reendothelialisierung verletzter Gefäße

Wildtyp C57BL6/J-Mäuse wurden ähnlich dem Protokoll zur Untersuchung der *in vivo* Adhäsion (s.o.) behandelt. EPCs (5x10¹/ml), die mit dem GPVI-CD133-Konstrukt (10 µg/ml9 oder beiden einzelnen Komponenten des Konstruktes zusammen (jeweils 10 µg/ml) 2 Stunden lang behandelt waren, wurden die Wunden der rechten Jugularvene geschlossen; die Tiere blieben anschließend am Leben. Nach zwei Wochen wurden die Tiere getötet und Proben aus der Halsschlagader entnommen. Regenerierende endothliale Zellen wurden durch Hämatoxilin-Eosin-(HE)-Färbung untersucht. Daneben wurde eine Elastica-von-Giesson-Färbung durchgeführt. Um zwischen lokalen Regenerations-Mechanismen und der durch die humanen EPCs induzierten Heildung zu unterscheiden, wurden in situ Hybridisierungen unter Verwendung einer alu-Sequenz, die spezifisch für humane Zellen ist, vorgenommen.

### Immunohistochemie von Paraffinschnitten

Immunohistochemie wurde unter Verwendung von Paraffinschnitten aus Maus-Gefäßen vorgenommen. Die Objektträger mit den Schnitten wurden mit Xylen entparaffinisiert (Carl Roth GmbH, Karlsruhe, Deutschland) und mit absteigenden Ethanol-Konzentrationen wieder rehydriert: 100%, 90%, 70%, 50 %. Anschließend wurden die Objektträger gründlich mit PBS gewaschen. Hierauf folgten jeweils 20-minütige Permeabilisierungs- und Blockierungsschritte mit PBS, das 0,1% Triton X-100 (Fluka Chemie, Buchs, Schweiz) und 1% BSA (Rinderserumalbumin) Lösung (Sigma Aldrich, St. Louis, USA) enthielt. Anschließend wurden die Objektträger mit dem primären Antikörper anti-vWF (2,5 µg/ml) (Chemicon, Temecula, USA) über 12 Stunden bei 4°C inkubiert. Danach wurde der sekundäre Ziegen- anti-Kaninchen-Antikörper (5µg/ml) (Molecular Probes/Invitrogen, Karlsruhe, Deutschland) und 0,1µg/ml DAPI ( Carl Roth GmbH, Karlsruhe Deutschland) für weitere 120 min bei Raumtemperatur hinzugegeben. Die Objektträger wurden gründlich mit PBS gewaschen, mit destilliertem Wasser abgewaschen, getrocknet und mit Kaisers Gelatine bedeckt (Merck, Darmstadt, Deutschland) und analysiert.

### Bestimmung der Neointima-Bildung

Männliche NOD/SCID Mäuse wurden gemäß einem Protokoll behandelt, das dem zuvor beschriebenen (siehe unter "Ligatur der Halsschlagader") ähnelt. Anstelle der Halsschlagaderligatur, wurde mittels eines Drahtes eine Verletzung herbeigeführt. Nach der Injektion von EPCs (5x10⁵/ml), welche zuvor 30 min mit dem GPVI-CD-133-Konstrukt (10 µg/ml) oder mit beiden einzelnen Komponenten des Konstrukts zusammen (jeweils 10 µg/ml) behandelt wurden, in die Schwanzvene, wurden die Wunden verschlossen und die Tiere am Leben erhalten. Nach 14 oder nach 21 Tagen wurden die Tiere getötet und die Halsschlagaderproben entnommen. Diese wurden in Paraffin-Blöcke eingebettet und vom proximalen bis zum distalen Ende in 5-µm-Schnitte geschnitten. 10 Abschnitte abwärts der Karotisgabel wurden für die Quantifizierung oder die Plaque-Bildung eingesetzt. Die Neointima-Bildung wurde im Querschnitt unter Verwendung einer Bildgebungs-Analyse-Software (Zeiss) bestimmt. Die Neointima wurde für jedes Tier als der Unterschied zwischen dem Bereich, der durch die interne elastische Lamina begrenzt ist, und dem Lumen-Bereich bestimmt. Auf ähnliche Weise wurde die Media bestimmt, und zwar als der Unterschied zwischen dem Bereich, der durch die interne elastische Lamina begrenzt ist, und der äußeren elastischen Lamina. Die Ergebnisse sind dargestellt als Neointima geteilt durch Media (Intima/Media-Verhältnis).

### Bestimmung des vaskulären Resistenzindex durch Duplex-Sonographie

Die Tiere wurden betäubt und die Halsschlagadern wurden mittels Duplex-Sonographie wie zuvor beschrieben (siehe Massberg et al., "A critical role of platelet adhesion in the initiation of atherosclerotic lesion formation", J. Exp. Med. 196:887-896 (2002)) visualisiert. Kurz gesagt wurde die maximale systolische Fließgeschwindigkeit V_{sys} und die endodiastolische Fließgeschwindigkeit V_{dia} bestimmt. Der Resistenzindex der Halsschlagader wurde als der Unterschied zwischen V_{sys} und V_{dia} getrennt durch Vsys bestimmt.

### Darstellung der Daten und Statistik

Die Vergleiche zwischen den Guppen-Mittelwerten wurden unter Verwendung der ANOVA Analyse oder des Student's t-Test durchgeführt. Die Daten werden als Mittelwerte ± Standard-Abweichung dargestellt. P<0,05 wurde als statistisch signifikant erachtet.

### Ergebnisse

Unter Verwendung von aus dem Knochenmark abgeleiteten humanen Stammzellen wurde zunächst die Adhäsion von EPCs an immobilisiertes Kollagen I in einem statischen Adhäsions-Assay sowie unter arteriellen Scher-Bedingungen in einem Flusskammer-Modell untersucht.

Bei dem statischen Adhäsions-Assay wurden 96-Well-Platten mit Kollagen I beschichtet und mit dem beschriebenen Produkt (10 µg/ml) für eine Stunde inkubiert. Anschließend wurden EPCs (CD34+ Zellen) hinzugefügt, 60 Minuten inkubiert und mit PBS gewaschen. Nach Inkubation der Kollagen-Oberfläche mit dem GPVI-CD133-Konstrukt ("GPVI-CD133", 10 µg/ml) war die Adhäsion um das 5-fache verstärkt um Vergleich zu Kollagen alleine (siehe **Fig. 2a****;** statisches Modell) und bei dem Flusskammer-Modell (2000 sec⁻¹) um das 10-fache **(****Fig. 2b****).** Kein Anstieg in der Adhäsion konnte bei einem Einsatz der zwei einzelnen Komponenten des Konstruktes beobachtet werden (jeweils 10 µg/ml) Die mittlere und die Standardabweichung von 4 unterschiedlichen Experimenten ist dargestellt. * bedeutet p= 0,021 in d Fig. 2a, und p= 0,025 in Fig. 2b. Dies bedeutet, dass das Konstrukt unter physiologischen Fluss-Bedingungen sogar effizienter ist. Ferner konnte in weiteren Versuchen gezeigt werden, dass die verstärkte Adhäsion der EPCs, die durch das GPVI-CD133-Konstrukt erreicht wurde, für immobilisiertes Kollagen spezifisch war im Vergleich zu Fibronectin (siehe **Fig. 2g****).**

In sämtlichen Figuren ist der Einsatz des Konstrukts mit "GPVI-CD133" bezeichnet, und der Einsatz der einzelnen Komponenten zusammen mit "GPVI + CD133".

Kürzlich wurde gezeigt, dass das Chemokin CXCL7 die Chemotaxis und die Adhäsion von EPCs an Komponenten der extrazelluläre Matrix bedeutend steigern kann. Diesbezüglich konnte in weiteren Versuchen gezeigt werden, dass das GPVI-CD133-Konstrukt noch wirksamer eine Rekrutierung der EPCs an immobilisiertes Kollagen bewirken kann als CXCL7 (siehe **Fig. 2h****).**

Nachdem die EPCs gebunden sind, werden sie in die Endothelschicht integriert, um zu der Wiederherstellung der Gefäßintegrität beizutragen. Daher wurde in anschließenden Versuchen gezeigt, dass nach Einsatzes des Konstruktes die Zellen nicht ihre Fähigkeit zur Differenzierung in Endothelzellen verlieren. Darüber hinaus konnte nach der Exposition gegenüber dem Konstrukt eine schnelle Änderung in der Morphologie beobachtet werden, weg vom kleinen, rundlichen Erscheinungsbild der EPCs hin zu einer eher Endothelzellen-Form. Nach Inkubation mit dem Konstrukt über 4 Tage hinweg, war das Potential der EPCs, endotheliale Kolonien zu bilden, signifikant verstärkt im Vergleich zu gleichen Versuchen, die mit den einzelnen Komponenten durchgeführt wurden (Negativ Kontrolle), und ähnlich zu der positiv Kontrolle Fibronectin (Siehe **Fig. 2c****;** Anzahl der Kolonien/10⁶ eingesetzter Zellen). Die mittlere ± Standardabweichung von 3 bis 5 unabhängigen Experimenten ist gezeigt. * entspricht p=9,001.

Darüber hinaus, konnte mit der Flusszytometrie gezeigt werden, dass die sich entwickelnden Zellen positiv für die Zellmarker CD31 und CD146 sind, die endotheliale Oberflächenmarker darstellen (siehe **Fig. 2d****).** Ferner konnten die Zellen positiv für die Marker vWF/Endoglin und Phalloidin gefärbt werden, die Marker reifer Endothelzellen darstellen. Der Nachweis erfolgte über Standard oder konkfokaler Immunfluoreszenz-Mikrosopie (siehe **Fig. 2e****).** Ferner konnten nach einer Inkubation mit dem Konstrukt für 8 Tage in der Transmissions-Elektronen-Mikroskopie eindeutig Weibel-Palade-Körperchen nachgewiesen werden, ein typsiches Merkmal reifer Endothelzellen (Fig. 1f; gezeigt mit ←, ≈ 300 nm x 60 nm; Vergrößerung x 80000). In unbehandelten CD34⁺ konnten keine Weibel-Palade Körperchen gefunden werden.

Um diese Ergebnisse in vivo zu bestätigen, wurde eine in vivo Fluoreszenz-Mikroskopie und ein Maus-Modell mit beschädigter Halsschlagader eingesetzt. Vor einem energischen Beschädigen der linken Halsschlagader, wurden mit DCF gefärbte EPS über die rechte Jugularvene injiziert, und die EPC-Adhäsion vor, nach 5 min, und nach 30 min nach der Verletzungs-Zufügung untersucht. Die Anzahl der adhärierenden EPCs war bedeutend angestiegen, wenn die Zellen zuvor mit dem GPVI-CD133-Konstrukt ("GPVI-CD133", 10 µg/ml) inkubiert wurden, im Vergleich zu den einzelnen Komponenten des Konstruktes ("GPVI + CD133", jeweils 10µg/ml) alleine (siehe **Fig. 3a****, b).** * bedeutet p=0,038 (feste Adhäsion), p= 0,025 (transiente Adhäsion).

Nach diesen Untersuchungen wurden die Halsschlagadern entfernt und mittels Zwei-Photonen-Mikroskopie untersucht. Eine offensichtliche Anreicherung grüner (DCFgefärbter) Zellen mit einem roten Nucelus konnte im Bereich der Denudaiton der luminalen Seite der Elastica interna beobachtet werden **(****Fig. 3c****).**

Um diese Ergebnisse auf ein dem Menschen vergleichbares System zu übertragen, wurden ein *ex vivo* Fluss-Modell eingesetzt. Hierzu wurden vor dem EPC-Einsatz und nach Perfusion für 2 Stunden die Gefäße von Schweinen mit einem Ballon-Katheter beschädigt. Anschließend wurden die Gefäße fixiert und die Rekrutierung der Zellen durch in situ Hybridisierung mit einer für Menschen spezifischen Sequenz untersucht. Die Rekrutierung der Stammzellen konnte durch den Einsatz des GPVI-CD133-Konstruktes bedeutend gesteigert werden, im Vergleich zu unverletzten Gefäßen (ungefär 50-fach, nicht gezeigt), zu verletzten Gefäßen, bei denen das Konstrukt nicht zum Einsatz kam (ungefähr 25-fach), oder wenn die beiden Komponenten des Konstrukts alleine eingesetzt wurden (ungefähr 10-fach) **(****Fig. 3d****).** * bedeutet p< 0,001 im Vergleich mit den beiden einzelnen Komponenten des Konstrukts.

Nach Exponierung der verletzten Mausarterien gegenüber EPCs, die mit dem bispezifischen Konstrukt behandelt waren, jedoch nicht nach einer Exponierung gegenüber den beiden einzelnen Komponenten alleine, über einen Zeitraum von acht Tagen *ex vivo* (Daten nicht gezeigt), oder über 14 Tage *in vivo,* konnte eine Gewinnung von Endothelzellen beobachtet werden **(****Fig. 3e****;** HE-Färbung). Um zwischen den Effekten, die durch die verabreichten Zellen verursacht wurden, und den Effekten, die durch lokale Regenerationsmechanismen verursacht wurden, zu unterscheiden, wurden immunodefiziente NOD/SCID Mäuse mit menschlichen EPCs behandelt. Anschließend wurden *in situ* Hybridisierungen unter Verwendung einer Alu Sonde durchgeführt. Diese spezifische Alu Sonde entspricht der Konsensussequenz menschlicher Alu Repeats und ermöglicht eine definitive Detektion menschlicher Zellen in Xenotransplantaten. Hierzu wurden die Mäuse 14 Tage nach Verletzung der Halsschlagader und nach Verabreichung von Zellen getötet. Intraluminale Zellen, die sich als positiv in der Färbung erwiesen, wurden als von menschlichen Zellen abgeleitete Zellen bestimmt. Diese Ergebnisse zeigen, dass die Neoendothelialisierung von Gefäßläsionen im Wesentlichen von extern injizierten EPCs herrührte.

Um ferner die funktionelle Bedeutung von GPVI-CD133 für die Gefäßneubildung *in vivo* einzuschätzen, wurde die Neointimabildung nach einer mit einem Draht zugefügten Verletzung untersucht. Zwei Wochen nach der Induktion der Verletzung wurde eine Tendenz in Richtung eines reduzierten Intima/Media-Verhältnisses und eines abgesenkten Gefäßresistenzindex beobachtet, ohne statistische Signifikanz, was durch Duplex-Sonographie bestimmt wurde (Daten nicht gezeigt). Auffallenderweise resultierte die Verabreichung von GPVI-CD133 in ein signifikant verkleinertes Intima/Media-Verhältnis 3 Wochen nach Induktion einer Verletzung der Halsschlagader, was auf den erwünschten Effekt bei der Gefäßneubildung hindeutet (Siehe **Fig. 3f****).** Auch bei diesen Versuchen wurde wieder entweder das Konstrukt (GPVI-CD133) oder die einzelnen Komponenten zusammen verabreicht (GPVI + CD133). In dem Diagramm von Fig. 3f bedeutet "*" p = 0.03 verglichen mit der Kontrolle; n = 5-6; pro Tier wurden 10 Abschnitte analysiert.

Zusammenfassend konnten die Erfinder somit zeigen, dass es mit dem erfindungsgemäßen Fusionsprotein (oben stehend und nachstehend auch als "Konstrukt" bezeichnet) EPCs (also CD34+-Stammzellen) an exponierte Kollagen-Oberflächen und verletzte Gefäße *in vitro, in vivo* und in humanen Gefäße angereichert werden konnten. Ferner konnten die Erfinder zeigen, dass eine längere Inkubation der Stammzellen mit dem Fusionsprotein die Differenzierung in reife Endothelzellen *in vitro* erreicht werden kann.

Für eine mögliche Therapie verletzter Gefäße/Gewebe bedeutet dies, dass das Fusionsprotein bzw. davon abgeleitete Varianten bspw. über einen Katheter in die entsprechenden Gefäße eingeführt werden kann, oder aber vor einer Verabreichung von Stammzellen mit diesen coinkubiert wird.

Die Ergebnisse zeigen, dass es mittels des erfindungsgemäßen Fusionsprotein möglich ist, zirkulierende endotheliale Vorläuferzellen an Kollagen-reiche Gefäßläsionen einzufangen, was sowohl durch *in vitro* als auch durch *in vivo* Versuche gezeigt werden konnte. Darüber hinaus verstärkte das Fusionsprotein die Differenzierung von endothelialen Vorläuferzellen (EPCs) in Endothelzellen und verstärkt die Reendothelialisierung von Gefäßläsionen.

## Patentansprüche

1. Bispezifisches Konstrukt, aufweisend:
(a) ein erstes Polypeptid, das an Kollagen bindet, und
(b) ein zweites Polypeptid, das an endotheliale Vorläuferzellen bindet,
**dadurch gekennzeichnet, dass** das erste Polypeptid der Kollagenrezeptor thrombocytäres Glycoprotein (GPVI) ist, oder funktionelle Fragmente davon, und dass das zweite Polypeptid ein Antikörper ist, der gegen CD133 gerichtet ist, oder funktionelle Fragmente davon.

2. Konstrukt nach Anspruch 1, **dadurch gekennzeichnet, dass** das erste Polypeptid einen extrazellulären Anteil von GPVI, oder funktionelle Fragmente davon, verbunden mit einem dimerisierenden Polypeptid aufweist.

3. Konstrukt nach Anspruch 2, **dadurch gekennzeichnet, dass** das dimerisierende Polypeptid eine Fc-Domäne eines Immunglobulins oder ein Fragment oder eine Variante davon aufweist, das bzw. die die Dimerisierungsfunktion der Fc-Domäne aufweist.

4. Konstrukt nach einem der Ansprüche 1 bis 3, **dadurch gekennzeichnet, dass** das erste Polypeptid die Aminosäuresequenz SEQ ID-Nr. 3 aus dem beigefügten Sequenzprotokoll aufweist.

5. Nucleinsäuremolekül, das für ein Konstrukt nach einem der Ansprüche 1 bis 4 kodiert.

6. Pharmazeutische und/oder diagnostische Zusammensetzung, die ein Konstrukt nach einem der Ansprüche 1 bis 4 aufweist, sowie ggf. zumindest einen pharmazeutisch akzeptablen Träger sowie ggf. weitere pharmazeutisch und/oder diagnostisch wirksame Stoffe aufweist.

7. Konstrukt nach einem der Ansprüche 1 bis 4 zur Verwendung in der Behandlung von Läsionen von Geweben und Gefäßen, bei welchen Kollagen exponiert ist.

8. Konstrukt nach Anspruch 7, **dadurch gekennzeichnet, dass** es zur Reendothelialisierung von Gefäßläsionen eingesetzt wird.

9. Konstrukt nach Anspruch 7 oder 8, **dadurch gekennzeichnet, dass** das die Gefäße und/oder Gewebe ausgewählt sind aus der Gruppe umfassend Herzkranzgefäße, Herzkranzgefäße, hirnversorgende Gefäße, extremitätenversorgende Gefäße, Bindegewebe, Knochen, sowie jedes Gefäß oder Gewebe, das Kollagen aufweist.

10. Konstrukt nach einem der Ansprüche 7 bis 9, **dadurch gekennzeichnet, dass** es zur Verabreichung über einen Ballonkatheter eingesetzt wird.

11. Verfahren zur Herstellung eines Konstrukt mit den folgenden Schritten:
(a) Bereitstellen (i) eines Polypeptids, das eine lösliche Form des Glycoproteins VI (GPVI) ist, und (ii) eines gegen CD133 gerichteten Antikörpers;
(b) Modifizieren der Aminogruppen von GPVI, und des Antikörpers mit einem vernetzendem Agens;
(c) Reduzieren von GPVI; und
(d) Konjugieren des reduzierten GPVI mit dem in Schritt (b) modifizierten Antikörper.

## Claims

1. A bispecific construct, comprising:
(a) a first polypeptide which binds to collagen, and
(b) a second polypeptide which binds to endothelial precursor cells,
**characterized in that** the first polypeptide is the collagen receptor thrombocytic glycoprotein VI (GPVI), or functional fragments thereof, and that the second polypeptide is an antibody directed against CD133, or functional fragments thereof.

2. The construct as claimed in claim 1, **characterized in that** the first polypeptide has an extracellular portion of GPVI, or functional fragments thereof, combined with a dimerizing polypeptide.

3. The construct as claimed in claim 2, **characterized in that** the dimerizing polypeptide has an Fc domain of an immunoglobulin or a fragment or a variant thereof which has the dimerization function of the Fc domain.

4. The construct as claimed in one of claims 1 to 3, **characterized in that** the first polypeptide has the amino acid sequence SEQ ID No. 3 from the attached sequence listing.

5. A nucleic acid molecule which codes for the fusion protein as claimed in one of claims 1 to 4.

6. A pharmaceutical and/or diagnostic composition which comprises the fusion protein according to one of claims 1 to 4, and optionally comprises at least one pharmaceutically acceptable carrier and optionally further pharmaceutically and/or diagnostically active substances.

7. Construct as claimed in one of claims 1 to 4 for use in the treatment of lesions of tissues and vessels where collagen is exposed.

8. The construct as claimed in claim 7, **characterized in that** it is employed for re-endothelialization of vessel lesions.

9. The construct as claimed in claim 7 or 8, **characterized in that** the vessels and/or tissue are chosen from the group including coronary vessels, vessels which supply the brain, vessels which supply the extremities, connective tissue, bone, and any vessel or tissue which contains collagen.

10. The construct as claimed in one of claims 7 to 9, **characterized in that** the composition is prepared for administration via a balloon catheter.

11. A process for the preparation of a construct with the following steps:
(a) providing (i) of a polypeptide that is a soluble form of glycoprotein VI (GPVI), and (ii) an antibody directed against CD133;
(b) modifying the amino groups of GPVI, and of the antibody with a crosslinking agent;
(c) reducing of GPVI; and
(d) conjugating the reduced GPVI with the antibody modified in step (b).

## Revendications

1. Construction bispécifique, comprenant :
(a) un premier polypeptide, qui se lie au collagène, et
(b) un deuxième polypeptide qui se lie aux cellules endothéliales précurseurs,
**caractérisée en ce que** le premier polypeptide est la glycoprotéine plaquettaire (GPVI) récepteur du collagène, ou des fragments fonctionnels de cette dernière, et que le deuxième polypeptide est un anticorps qui est dirigé contre le CD133, ou des fragments fonctionnels de ce dernier.

2. Construction selon la revendication 1, **caractérisée en ce que** le premier polypeptide comprend une portion extracellulaire du GPVI, ou des fragments fonctionnels de ce dernier, reliée à un polypeptide de dimérisation.

3. Construction selon la revendication 2, **caractérisée en ce que** le polypeptide de dimérisation comprend un domaine Fc d'une immunoglobuline ou un fragment ou un variant de ce dernier, qui comprend la fonction de dimérisation du domaine Fc.

4. Construction selon l'une des revendications 1 à 3, **caractérisée en ce que** le premier polypeptide présente la séquence d'acides aminés SEQ ID N° 3 de la liste de séquences ci-jointe.

5. Molécule d'acide nucléique qui code pour une construction selon l'une des revendications 1 à 4.

6. Composition pharmaceutique et/ou diagnostique, qui comprend une construction selon l'une des revendications 1 à 4, ainsi qu'éventuellement au moins un support pharmaceutiquement acceptable, ainsi qu'éventuellement des principes actifs supplémentaires, efficaces d'un point de vue pharmaceutique et/ou diagnostique.

7. Construction selon l'une des revendications 1 à 4 pour utilisation dans le traitement de lésions de tissus et de vaisseaux dans lesquelles il y a exposition du collagène.

8. Construction selon la revendication 7, **caractérisée en ce qu'**elle est utilisée pour la réendothélialisation de lésions vasculaires.

9. Construction selon la revendication 7 ou 8, **caractérisée en ce que** les vaisseaux et/ou tissus sont choisis dans le groupe comprenant les vaisseaux coronariens, les vaisseaux coronariens, les vaisseaux alimentant le cerveau, les vaisseaux alimentant les extrémités, les tissus conjonctifs, les os, ainsi que tout vaisseau ou tissu qui comprend du collagène.

10. Construction selon l'une des revendications 7 à 9, **caractérisée en ce qu'**elle est utilisé pour une administration par l'intermédiaire d'un cathéter à ballonnet.

11. Procédé de fabrication d'une construction, comportant les étapes suivantes :
(a) mise à disposition (i) d'un polypeptide qui est une forme soluble de la glycoprotéine VI (GPVI), et (ii) d'un anticorps dirigé contre le CD133 ;
(b) modification des groupes amino du GPVI et de l'anticorps à l'aide d'un agent réticulant ;
(c) réduction du GPVI ; et
(d) conjugaison du GPVI réduit et de l'anticorps modifié dans l'étape (b).


### SEQUENZPROTOKOLL

<110> Eberhard-Karls-Universität Tübingen, Universitätsklinikum
<120> Bispezifisches Fusionsprotein mit therapeutischem und diagnostischem Potential
<130> 5402P364
<160> 7
<170> PatentIn version 3.1
<210> 1
   <211> 339
   <212> PRT
   <213> Homo sapiens
<400> 1
   Met Ser Pro Ser Pro Thr Ala Leu Phe Cys Leu Gly Leu Cys Leu Gly
   1               5                   10                  15
   Arg Val Pro Ala Gln Ser Gly Pro Leu Pro Lys Pro Ser Leu Gln Ala
               20                  25                  30
   Leu Pro Ser Ser Leu Val Pro Leu Glu Lys Pro Val Thr Leu Arg Cys
           35                  40                  45
   Gln Gly Pro Pro Gly Val Asp Leu Tyr Arg Leu Glu Lys Leu Ser Ser
       50                  55                  60
   Ser Arg Tyr Gln Asp Gln Ala Val Leu Phe Ile Pro Ala Met Lys Arg
   65                  70                  75                  80
   Ser Leu Ala Gly Arg Tyr Arg Cys Ser Tyr Gln Asn Gly Ser Leu Trp
                   85                  90                  95
   Ser Leu Pro Ser Asp Gln Leu Glu Leu Val Ala Thr Gly Val Phe Ala
               100                 105                 110
   Lys Pro Ser Leu Ser Ala Gln Pro Gly Pro Ala Val Ser Ser Gly Gly
           115                 120                 125
   Asp Val Thr Leu Gln Cys Gln Thr Arg Tyr Gly Phe Asp Gln Phe Ala
       130                 135                 140
   Leu Tyr Lys Glu Gly Asp Pro Ala Pro Tyr Lys Asn Pro Glu Arg Trp
   145                 150                 155                 160
   Tyr Arg Ala Ser Phe Pro Ile Ile Thr Val Thr Ala Ala His Ser Gly
                   165                 170                 175
   Thr Tyr Arg Cys Tyr Ser Phe Ser Ser Arg Asp Pro Tyr Leu Trp Ser
               180                 185                 190
   Ala Pro Ser Asp Pro Leu Glu Leu Val Val Thr Gly Thr Ser Val Thr
           195                 200                 205
   Pro Ser Arg Leu Pro Thr Glu Pro Pro Ser Ser Val Ala Glu Phe Ser
       210                 215                 220
   Glu Ala Thr Ala Glu Leu Thr Val Ser Phe Thr Asn Lys Val Phe Thr
   225                 230                 235                 240
   Thr Glu Thr Ser Arg Ser Ile Thr Thr Ser Pro Lys Glu Ser Asp Ser
                   245                 250                 255
   Pro Ala Gly Pro Ala Arg Gln Tyr Tyr Thr Lys Gly Asn Leu Val Arg
   260 265 270
   Ile Cys Leu Gly Ala Val Ile Leu Ile Ile Leu Ala Gly Phe Leu Ala
   275 280 285
   Glu Asp Trp His Ser Arg Arg Lys Arg Leu Arg His Arg Gly Arg Ala
   290 295 300
   Val Gln Arg Pro Leu Pro Pro Leu Pro Pro Leu Pro Gln Thr Arg Lys
   305 310 315 320
   Ser His Gly Gly Gln Asp Gly Gly Arg Gln Asp Val His Ser Arg Gly
   325 330 335
   Leu Cys Ser
<210> 2
   <211> 1020
   <212> DNA
   <213> Homo sapiens
<400> 2
   atgtctccat ccccgaccgc cctcttctgt cttgggctgt gtctggggcg tgtgccagcg 60
   cagagtggac cgctccccaa gccctccctc caggctctgc ccagctccct ggtgcccctg 120
   gagaagccag tgaccctccg gtgccaggga cctccgggcg tggacctgta ccgcctggag 180
   aagctgagtt ccagcaggta ccaggatcag gcagtcctct tcatcccggc catgaagaga 240
   agtctggctg gacgctaccg ctgctcctac cagaacggaa gcctctggtc cctgcccagc 300
   gaccagctgg agctcgttgc cacgggagtt tttgccaaac cctcgctctc agcccagccc 360
   ggcccggcgg tgtcgtcagg aggggacgta accctacagt gtcagactcg gtatggcttt     420
   gaccaatttg ctctgtacaa ggaaggggac cctgcgccct acaagaatcc cgagagatgg     480
   taccgggcta gtttccccat catcacggtg accgccgccc acagcggaac ctaccgatgc     540
   tacagcttct ccagcaggga cccatacctg tggtcggccc ccagcgaccc cctggagctt     600
   gtggtcacag gaacctctgt gacccccagc cggttaccaa cagaaccacc ttcctcggta     660
   gcagaattct cagaagccac cgctgaactg accgtctcat tcacaaacaa agtcttcaca     720
   actgagactt ctaggagtat caccaccagt ccaaaggagt cagactctcc agctggtcct     780
   gcccgccagt actacaccaa gggcaacctg gtccggatat gcctcggggc tgtgatccta     840
   ataatcctgg cggggtttct ggcagaggac tggcacagcc ggaggaagcg cctgcggcac     900
   aggggcaggg ctgtgcagag gccgcttccg cccctcccgc ccctcccgca gacccggaaa     960
   tcacacgggg gtcaggatgg aggccgacag gatgttcaca gccgcgggtt atgttcatga    1020
<210> 3
   <211> 247
   <212> PRT
   <213> Homo sapiens
<400> 3
   Gln Ser Gly Pro Leu Pro Lys Pro Ser Leu Gln Ala Leu Pro Ser Ser
   1               5                   10                  15
   Leu Val Pro Leu Glu Lys Pro Val Thr Leu Arg Cys Gln Gly Pro Pro
               20                  25                  30
   Gly Val Asp Leu Tyr Arg Leu Glu Lys Leu Ser Ser Ser Arg Tyr Gln
           35                  40                  45
   Asp Gln Ala Val Leu Phe Ile Pro Ala Met Lys Arg Ser Leu Ala Gly
   50 55 60
   Arg Tyr Arg Cys Ser Tyr Gln Asn Gly Ser Leu Trp Ser Leu Pro Ser
   65 70 75 80
   Asp Gln Leu Glu Leu Val Ala Thr Gly Val Phe Ala Lys Pro Ser Leu
   85 90 95
   Ser Ala Gln Pro Gly Pro Ala Val Ser Ser Gly Gly Asp Val Thr Leu
   100 105 110
   Gln Cys Gln Thr Arg Tyr Gly Phe Asp Gln Phe Ala Leu Tyr Lys Glu
   115 120 125
   Gly Asp Pro Ala Pro Tyr Lys Asn Pro Glu Arg Trp Tyr Arg Ala Ser
   130 135 140
   Phe Pro Ile Ile Thr Val Thr Ala Ala His Ser Gly Thr Tyr Arg Cys
   145 150 155 160
   Tyr Ser Phe Ser Ser Arg Asp Pro Tyr Leu Trp Ser Ala Pro Ser Asp
   165 170 175
   Pro Leu Glu Leu Val Val Thr Gly Thr Ser Val Thr Pro Ser Arg Leu
   180 185 190
   Pro Thr Glu Pro Pro Ser Ser Val Ala Glu Phe Ser Glu Ala Thr Ala
   195 200 205
   Glu Leu Thr Val Ser Phe Thr Asn Lys Val Phe Thr Thr Glu Thr Ser
   210 215 220
   Arg Ser Ile Thr Thr Ser Pro Lys Glu Ser Asp Ser Pro Ala Gly Pro
   225 230 235 240
   Ala Arg Gln Tyr Tyr Thr Lys
   245
<210> 4
   <211> 913
   <212> PRT
   <213> Homo sapiens
<400> 4
   Met Gly Pro Glu Ala Leu Ser Ser Leu Leu Leu Leu Leu Leu Val Ala
   1 5 10 15
   Ser Gly Asp Ala Asp Met Lys Gly His Phe Asp Pro Ala Lys Cys Arg
   20 25 30
   Tyr Ala Leu Gly Met Gln Asp Arg Thr Ile Pro Asp Ser Asp Ile Ser
   35 40 45
   Ala Ser Ser Ser Trp Ser Asp Ser Thr Ala Ala Arg His Ser Arg Leu
   50 55 60
   Glu Ser Ser Asp Gly Asp Gly Ala Trp Cys Pro Ala Gly Ser Val Phe
   65 70 75 80
   Pro Lys Glu Glu Glu Tyr Leu Gln Val Asp Leu Gln Arg Leu His Leu
   85 90 95
   Val Ala Leu Val Gly Thr Gln Gly Arg His Ala Gly Gly Leu Gly Lys
   100 105 110
   Glu Phe Ser Arg Ser Tyr Arg Leu Arg Tyr Ser Arg Asp Gly Arg Arg
   115 120 125
   Trp Met Gly Trp Lys Asp Arg Trp Gly Gln Glu Val Ile Ser Gly Asn
   130 135 140
   Glu Asp Pro Glu Gly Val Val Leu Lys Asp Leu Gly Pro Pro Met Val
   145 150 155 160
   Ala Arg Leu Val Arg Phe Tyr Pro Arg Ala Asp Arg Val Met Ser Val
   165 170 175
   Cys Leu Arg Val Glu Leu Tyr Gly Cys Leu Trp Arg Asp Gly Leu Leu
   180 185 190
   Ser Tyr Thr Ala Pro Val Gly Gln Thr Met Tyr Leu Ser Glu Ala Val
   195 200 205
   Tyr Leu Asn Asp Ser Thr Tyr Asp Gly His Thr Val Gly Gly Leu Gln
   210 215 220
   Tyr Gly Gly Leu Gly Gln Leu Ala Asp Gly Val Val Gly Leu Asp Asp
   225 230 235 240
   Phe Arg Lys Ser Gln Glu Leu Arg Val Trp Pro Gly Tyr Asp Tyr Val
   245 250 255
   Gly Trp Ser Asn His Ser Phe Ser Ser Gly Tyr Val Glu Met Glu Phe
   260 265 270
   Glu Phe Asp Arg Leu Arg Ala Phe Gln Ala Met Gln Val His Cys Asn
   275 280 285
   Asn Met His Thr Leu Gly Ala Arg Leu Pro Gly Gly Val Glu Cys Arg
   290 295 300
   Phe Arg Arg Gly Pro Ala Met Ala Trp Glu Gly Glu Pro Met Arg His
   305 310 315 320
   Asn Leu Gly Gly Asn Leu Gly Asp Pro Arg Ala Arg Ala Val Ser Val
   325 330 335
   Pro Leu Gly Gly Arg Val Ala Arg Phe Leu Gln Cys Arg Phe Leu Phe
   340 345 350
   Ala Gly Pro Trp Leu Leu Phe Ser Glu Ile Ser Phe Ile Ser Asp Val
   355 360 365
   Val Asn Asn Ser Ser Pro Ala Leu Gly Gly Thr Phe Pro Pro Ala Pro
   370 375 380
   Trp Trp Pro Pro Gly Pro Pro Pro Thr Asn Phe Ser Ser Leu Glu Leu
   385 390 395 400
   Glu Pro Arg Gly Gln Gln Pro Val Ala Lys Ala Glu Gly Ser Pro Thr
   405 410 415
   Ala Ile Leu Ile Gly Cys Leu Val Ala Ile Ile Leu Leu Leu Leu Leu
   420 425 430
   Ile Ile Ala Leu Met Leu Trp Arg Leu His Trp Arg Arg Leu Leu Ser
           435                 440                 445
   Lys Ala Glu Arg Arg Val Leu Glu Glu Glu Leu Thr Val His Leu Ser
       450                 455                 460
   Val Pro Gly Asp Thr Ile Leu Ile Asn Asn Arg Pro Gly Pro Arg Glu
   465                 470                 475                 480
   Pro Pro Pro Tyr Gln Glu Pro Arg Pro Arg Gly Asn Pro Pro His Ser
                   485                 490                 495
   Ala Pro Cys Val Pro Asn Gly Ser Ala Leu Leu Leu Ser Asn Pro Ala
               500                 505                 510
   Tyr Arg Leu Leu Leu Ala Thr Tyr Ala Arg Pro Pro Arg Gly Pro Gly
           515                 520                 525
   Pro Pro Thr Pro Ala Trp Ala Lys Pro Thr Asn Thr Gln Ala Tyr Ser
       530                 535                 540
   Gly Asp Tyr Met Glu Pro Glu Lys Pro Gly Ala Pro Leu Leu Pro Pro
   545                 550                 555                 560
   Pro Pro Gln Asn Ser Val Pro His Tyr Ala Glu Ala Asp Ile Val Thr
                   565                 570                 575
   Leu Gln Gly Val Thr Gly Gly Asn Thr Tyr Ala Val Pro Ala Leu Pro
               580                 585                 590
   Pro Gly Ala Val Gly Asp Gly Pro Pro Arg Val Asp Phe Pro Arg Ser
           595                 600                 605
   Arg Leu Arg Phe Lys Glu Lys Leu Gly Glu Gly Gln Phe Gly Glu Val
       610                 615                 620
   His Leu Cys Glu Val Asp Ser Pro Gln Asp Leu Val Ser Leu Asp Phe
   625                 630                 635                 640
   Pro Leu Asn Val Arg Lys Gly His Pro Leu Leu Val Ala Val Lys Ile
                   645                 650                 655
   Leu Arg Pro Asp Ala Thr Lys Asn Ala Arg Asn Asp Phe Leu Lys Glu
               660                 665                 670
   Val Lys Ile Met Ser Arg Leu Lys Asp Pro Asn Ile Ile Arg Leu Leu
           675                 680                 685
   Gly Val Cys Val Gln Asp Asp Pro Leu Cys Met Ile Thr Asp Tyr Met
       690                 695                 700
   Glu Asn Gly Asp Leu Asn Gln Phe Leu Ser Ala His Gln Leu Glu Asp
   705                 710                 715                 720
   Lys Ala Ala Glu Gly Ala Pro Gly Asp Gly Gln Ala Ala Gln Gly Pro
                   725                 730                 735
   Thr Ile Ser Tyr Pro Met Leu Leu His Val Ala Ala Gln Ile Ala Ser
               740                 745                 750
   Gly Met Arg Tyr Leu Ala Thr Leu Asn Phe Val His Arg Asp Leu Ala
           755                 760                 765
   Thr Arg Asn Cys Leu Val Gly Glu Asn Phe Thr Ile Lys Ile Ala Asp
   770 775 780
   Phe Gly Met Ser Arg Asn Leu Tyr Ala Gly Asp Tyr Tyr Arg Val Gln
   785 790 795 800
   Gly Arg Ala Val Leu Pro Ile Arg Trp Met Ala Trp Glu Cys Ile Leu
   805 810 815
   Met Gly Lys Phe Thr Thr Ala Ser Asp Val Trp Ala Phe Gly Val Thr
   820 825 830
   Leu Trp Glu Val Leu Met Leu Cys Arg Ala Gln Pro Phe Gly Gln Leu
   835 840 845
   Thr Asp Glu Gln Val Ile Glu Asn Ala Gly Glu Phe Phe Arg Asp Gln
   850 855 860
   Gly Arg Gln Val Tyr Leu Ser Arg Pro Pro Ala Cys Pro Gln Gly Leu
   865 870 875 880
   Tyr Glu Leu Met Leu Arg Cys Trp Ser Arg Glu Ser Glu Gln Arg Pro
   885 890 895
   Pro Phe Ser Gln Leu His Arg Phe Leu Ala Glu Asp Ala Leu Asn Thr
   900 905 910
   Val
<210> 5
   <211> 398
   <212> PRT
   <213> Homo sapiens
<400> 5
   Asp Ala Asp Met Lys Gly His Phe Asp Pro Ala Lys Cys Arg Tyr Ala
   1               5                   10                  15
   Leu Gly Met Gln Asp Arg Thr Ile Pro Asp Ser Asp Ile Ser Ala Ser
               20                  25                  30
   Ser Ser Trp Ser Asp Ser Thr Ala Ala Arg His Ser Arg Leu Glu Ser
           35                  40                  45
   Ser Asp Gly Asp Gly Ala Trp Cys Pro Ala Gly Ser Val Phe Pro Lys
       50                  55                  60
   Glu Glu Glu Tyr Leu Gln Val Asp Leu Gln Arg Leu His Leu Val Ala
   65                  70                  75                  80
   Leu Val Gly Thr Gln Gly Arg His Ala Gly Gly Leu Gly Lys Glu Phe
                   85                  90                  95
   Ser Arg Ser Tyr Arg Leu Arg Tyr Ser Arg Asp Gly Arg Arg Trp Met
               100                 105                 110
   Gly Trp Lys Asp Arg Trp Gly Gln Glu Val Ile Ser Gly Asn Glu Asp
           115                 120                 125
   Pro Glu Gly Val Val Leu Lys Asp Leu Gly Pro Pro Met Val Ala Arg
       130                 135                 140
   Leu Val Arg Phe Tyr Pro Arg Ala Asp Arg Val Met Ser Val Cys Leu
   145                 150                 155                 160
   Arg Val Glu Leu Tyr Gly Cys Leu Trp Arg Asp Gly Leu Leu Ser Tyr
                   165                 170                 175
   Thr Ala Pro Val Gly Gln Thr Met Tyr Leu Ser Glu Ala Val Tyr Leu
               180                 185                 190
   Asn Asp Ser Thr Tyr Asp Gly His Thr Val Gly Gly Leu Gln Tyr Gly
           195                 200                 205
   Gly Leu Gly Gln Leu Ala Asp Gly Val Val Gly Leu Asp Asp Phe Arg
       210                 215                 220
   Lys Ser Gln Glu Leu Arg Val Trp Pro Gly Tyr Asp Tyr Val Gly Trp
   225                 230                 235                 240
   Ser Asn His Ser Phe Ser Ser Gly Tyr Val Glu Met Glu Phe Glu Phe
                   245                 250                 255
   Asp Arg Leu Arg Ala Phe Gln Ala Met Gln Val His Cys Asn Asn Met
               260                 265                 270
   His Thr Leu Gly Ala Arg Leu Pro Gly Gly Val Glu Cys Arg Phe Arg
           275                 280                 285
   Arg Gly Pro Ala Met Ala Trp Glu Gly Glu Pro Met Arg His Asn Leu
       290                 295                 300
   Gly Gly Asn Leu Gly Asp Pro Arg Ala Arg Ala Val Ser Val Pro Leu
   305                 310                 315                 320
   Gly Gly Arg Val Ala Arg Phe Leu Gln Cys Arg Phe Leu Phe Ala Gly
   325 330 335
   Pro Trp Leu Leu Phe Ser Glu Ile Ser Phe Ile Ser Asp Val Val Asn
   340 345 350
   Asn Ser Ser Pro Ala Leu Gly Gly Thr Phe Pro Pro Ala Pro Trp Trp
   355 360 365
   Pro Pro Gly Pro Pro Pro Thr Asn Phe Ser Ser Leu Glu Leu Glu Pro
   370 375 380
   Arg Gly Gln Gln Pro Val Ala Lys Ala Glu Gly Ser Pro Thr
   385 390 395
<210> 6
   <211> 855
   <212> PRT
   <213> Homo sapiens
<400> 6
   Met Ile Leu Ile Pro Arg Met Leu Leu Val Leu Phe Leu Leu Leu Pro
   1 5 10 15
   Ile Leu Ser Ser Ala Lys Ala Gln Val Asn Pro Ala Ile Cys Arg Tyr
   20 25 30
   Pro Leu Gly Met Ser Gly Gly Gln Ile Pro Asp Glu Asp Ile Thr Ala
   35 40 45
   Ser Ser Gln Trp Ser Glu Ser Thr Ala Ala Lys Tyr Gly Arg Leu Asp
   50 55 60
   Ser Glu Glu Gly Asp Gly Ala Trp Cys Pro Glu Ile Pro Val Glu Pro
   65 70 75 80
   Asp Asp Leu Lys Glu Phe Leu Gln Ile Asp Leu His Thr Leu His Phe
   85 90 95
   Ile Thr Leu Val Gly Thr Gln Gly Arg His Ala Gly Gly His Gly Ile
   100 105 110
   Glu Phe Ala Pro Met Tyr Lys Ile Asn Tyr Ser Arg Asp Gly Thr Arg
   115 120 125
   Trp Ile Ser Trp Arg Asn Arg His Gly Lys Gln Val Leu Asp Gly Asn
   130 135 140
   Ser Asn Pro Tyr Asp Ile Phe Leu Lys Asp Leu Glu Pro Pro Ile Val
   145 150 155 160
   Ala Arg Phe Val Arg Phe Ile Pro Val Thr Asp His Ser Met Asn Val
   165 170 175
   Cys Met Arg Val Glu Leu Tyr Gly Cys Val Trp Leu Asp Gly Leu Val
   180 185 190
   Ser Tyr Asn Ala Pro Ala Gly Gln Gln Phe Val Leu Pro Gly Gly Ser
   195 200 205
   Ile Ile Tyr Leu Asn Asp Ser Val Tyr Asp Gly Ala Val Gly Tyr Ser
   210 215 220
   Met Thr Glu Gly Leu Gly Gln Leu Thr Asp Gly Val Ser Gly Leu Asp
   225 230 235 240
   Asp Phe Thr Gln Thr His Glu Tyr His Val Trp Pro Gly Tyr Asp Tyr
   245 250 255
   Val Gly Trp Arg Asn Glu Ser Ala Thr Asn Gly Tyr Ile Glu Ile Met
   260 265 270
   Phe Glu Phe Asp Arg Ile Arg Asn Phe Thr Thr Met Lys Val His Cys
   275 280 285
   Asn Asn Met Phe Ala Lys Gly Val Lys Ile Phe Lys Glu Val Gln Cys
   290 295 300
   Tyr Phe Arg Ser Glu Ala Ser Glu Trp Glu Pro Asn Ala Ile Ser Phe
   305 310 315 320
   Pro Leu Val Leu Asp Asp Val Asn Pro Ser Ala Arg Phe Val Thr Val
   325 330 335
   Pro Leu His His Arg Met Ala Ser Ala Ile Lys Cys Gln Tyr His Phe
   340 345 350
   Ala Asp Thr Trp Met Met Phe Ser Glu Ile Thr Phe Gln Ser Asp Ala
   355 360 365
   Ala Met Tyr Asn Asn Ser Glu Ala Leu Pro Thr Ser Pro Met Ala Pro
   370 375 380
   Thr Thr Tyr Asp Pro Met Leu Lys Val Asp Asp Ser Asn Thr Arg Ile
   385 390 395 400
   Leu Ile Gly Cys Leu Val Ala Ile Ile Phe Ile Leu Leu Ala Ile Ile
   405 410 415
   Val Ile Ile Leu Trp Arg Gln Phe Trp Gln Lys Met Leu Glu Lys Ala
   420 425 430
   Ser Arg Arg Met Leu Asp Asp Glu Met Thr Val Ser Leu Ser Leu Pro
   435 440 445
   Ser Asp Ser Ser Met Phe Asn Asn Asn Arg Ser Ser Ser Pro Ser Glu
   450 455 460
   Gln Gly Ser Asn Ser Thr Tyr Asp Arg Ile Phe Pro Leu Arg Pro Asp
   465 470 475 480
   Tyr Gln Glu Pro Ser Arg Leu Ile Arg Lys Leu Pro Glu Phe Ala Pro
   485 490 495
   Gly Glu Glu Glu Ser Gly Cys Ser Gly Val Val Lys Pro Val Gln Pro
   500 505 510
   Ser Gly Pro Glu Gly Val Pro His Tyr Ala Glu Ala Asp Ile Val Asn
   515 520 525
   Leu Gln Gly Val Thr Gly Gly Asn Thr Tyr Ser Val Pro Ala Val Thr
   530 535 540
   Met Asp Leu Leu Ser Gly Lys Asp Val Ala Val Glu Glu Phe Pro Arg
   545 550 555 560
   Lys Leu Leu Thr Phe Lys Glu Lys Leu Gly Glu Gly Gln Phe Gly Glu
   565 570 575
   Val His Leu Cys Glu Val Glu Gly Met Glu Lys Phe Lys Asp Lys Asp
   580 585 590
   Phe Ala Leu Asp Val Ser Ala Asn Gln Pro Val Leu Val Ala Val Lys
   595 600 605
   Met Leu Arg Ala Asp Ala Asn Lys Asn Ala Arg Asn Asp Phe Leu Lys
   610 615 620
   Glu Ile Lys Ile Met Ser Arg Leu Lys Asp Pro Asn Ile Ile His Leu
   625 630 635 640
   Leu Ser Val Cys Ile Thr Asp Asp Pro Leu Cys Met Ile Thr Glu Tyr
   645 650 655
   Met Glu Asn Gly Asp Leu Asn Gln Phe Leu Ser Arg His Glu Pro Pro
   660 665 670
   Asn Ser Ser Ser Ser Asp Val Arg Thr Val Ser Tyr Thr Asn Leu Lys
   675 680 685
   Phe Met Ala Thr Gln Ile Ala Ser Gly Met Lys Tyr Leu Ser Ser Leu
   690 695 700
   Asn Phe Val His Arg Asp Leu Ala Thr Arg Asn Cys Leu Val Gly Lys
   705 710 715 720
   Asn Tyr Thr Ile Lys Ile Ala Asp Phe Gly Met Ser Arg Asn Leu Tyr
   725 730 735
   Ser Gly Asp Tyr Tyr Arg Ile Gln Gly Arg Ala Val Leu Pro Ile Arg
   740 745 750
   Trp Met Ser Trp Glu Ser Ile Leu Leu Gly Lys Phe Thr Thr Ala Ser
   755 760 765
   Asp Val Trp Ala Phe Gly Val Thr Leu Trp Glu Thr Phe Thr Phe Cys
   770 775 780
   Gln Glu Gln Pro Tyr Ser Gln Leu Ser Asp Glu Gln Val Ile Glu Asn
   785 790 795 800
   Thr Gly Glu Phe Phe Arg Asp Gln Gly Arg Gln Thr Tyr Leu Pro Gln
   805 810 815
   Pro Ala Ile Cys Pro Asp Ser Val Tyr Lys Leu Met Leu Ser Cys Trp
   820 825 830
   Arg Arg Asp Thr Lys Asn Arg Pro Ser Phe Gln Glu Ile His Leu Leu
   835 840 845
   Leu Leu Gln Gln Gly Asp Glu
   850 855
<210> 7
   <211> 378
   <212> PRT
   <213> Homo sapiens
<400> 7
   Lys Ala Gln Val Asn Pro Ala Ile Cys Arg Tyr Pro Leu Gly Met Ser
   1 5 10 15
   Gly Gly Gln Ile Pro Asp Glu Asp Ile Thr Ala Ser Ser Gln Trp Ser
   20 25 30
   Glu Ser Thr Ala Ala Lys Tyr Gly Arg Leu Asp Ser Glu Glu Gly Asp
   35 40 45
   Gly Ala Trp Cys Pro Glu Ile Pro Val Glu Pro Asp Asp Leu Lys Glu
   50 55 60
   Phe Leu Gln Ile Asp Leu His Thr Leu His Phe Ile Thr Leu Val Gly
   65 70 75 80
   Thr Gln Gly Arg His Ala Gly Gly His Gly Ile Glu Phe Ala Pro Met
   85 90 95
   Tyr Lys Ile Asn Tyr Ser Arg Asp Gly Thr Arg Trp Ile Ser Trp Arg
   100 105 110
   Asn Arg His Gly Lys Gln Val Leu Asp Gly Asn Ser Asn Pro Tyr Asp
   115 120 125
   Ile Phe Leu Lys Asp Leu Glu Pro Pro Ile Val Ala Arg Phe Val Arg
   130 135 140
   Phe Ile Pro Val Thr Asp His Ser Met Asn Val Cys Met Arg Val Glu
   145 150 155 160
   Leu Tyr Gly Cys Val Trp Leu Asp Gly Leu Val Ser Tyr Asn Ala Pro
   165 170 175
   Ala Gly Gln Gln Phe Val Leu Pro Gly Gly Ser Ile Ile Tyr Leu Asn
   180 185 190
   Asp Ser Val Tyr Asp Gly Ala Val Gly Tyr Ser Met Thr Glu Gly Leu
   195 200 205
   Gly Gln Leu Thr Asp Gly Val Ser Gly Leu Asp Asp Phe Thr Gln Thr
   210 215 220
   His Glu Tyr His Val Trp Pro Gly Tyr Asp Tyr Val Gly Trp Arg Asn
   225 230 235 240
   Glu Ser Ala Thr Asn Gly Tyr Ile Glu Ile Met Phe Glu Phe Asp Arg
   245 250 255
   Ile Arg Asn Phe Thr Thr Met Lys Val His Cys Asn Asn Met Phe Ala
   260 265 270
   Lys Gly Val Lys Ile Phe Lys Glu Val Gln Cys Tyr Phe Arg Ser Glu
   275 280 285
   Ala Ser Glu Trp Glu Pro Asn Ala Ile Ser Phe Pro Leu Val Leu Asp
   290 295 300
   Asp Val Asn Pro Ser Ala Arg Phe Val Thr Val Pro Leu His His Arg
   305 310 315 320
   Met Ala Ser Ala Ile Lys Cys Gln Tyr His Phe Ala Asp Thr Trp Met
   325 330 335
   Met Phe Ser Glu Ile Thr Phe Gln Ser Asp Ala Ala Met Tyr Asn Asn
   340 345 350
   Ser Glu Ala Leu Pro Thr Ser Pro Met Ala Pro Thr Thr Tyr Asp Pro
   355 360 365
   Met Leu Lys Val Asp Asp Ser Asn Thr Arg
   370 375